# EUROPÄISCHE PATENTSCHRIFT

(11) **EP 2 220 493 B1**
(45) Veröffentlichungstag und Bekanntmachung des Hinweises auf die Patenterteilung: **28.01.2015**
(21) Anmeldenummer: 08869063.1
(22) Anmeldetag: 16.12.2008
(51) Int. Cl.: C07K 14/005, A61K 39/00, G01N 33/576

(54) **NEUE OBERFLÄCHEN-PROTEIN-MUTANTE DES HEPATITIS B VIRUS HBV SURFACE ANTIGEN (HBSAG)**
NOVEL SURFACE PROTEIN MUTANTS OF THE HEPATITIS B VIRUS HBV SURFACE ANTIGEN (HBSAG)
NOUVELLE MUTANTE DE PROTÉINE DE SURFACE DE L'ANTIGÈNE DE SURFACE DU VIRUS HBV DE L'HÉPATITE B (HBSAG)

(30) Priorität: 21.12.2007 DE 102007062962
(43) Veröffentlichungstag der Anmeldung: 25.08.2010
(73) Patentinhaber: Siemens Healthcare Diagnostics Products GmbH, 35041 Marburg (DE)
(72) Erfinder: BUSSFELD, Delia, 35043 Marburg (DE); ENDRES, Anne-Sophie, 10115 Berlin (DE); MEISEL, Helga, 13127 Berlin (DE); WEIK, Michael, 35091 Cölbe-Schönstadt (DE)
(86) Internationale Anmeldenummer: PCT/EP2008/010708
(87) Internationale Veröffentlichungsnummer: WO 2009/083136

(56) Entgegenhaltungen:
- WO-A-03/087351
- WO-A-2004/113370
- WO-A-2005/042733
- US-A1- 2007 042 356
- MEISEL H ET AL: "Transmission of hepatitis B virus 2 months prior to hepatitis surface antigen positivity of donor blood." TRANSFUSION MEDICINE AND HEMOTHERAPY, Bd. 30, Nr. 5, 2003, Seiten 228-231, XP008105773 ISSN: 1660-3796
- MARSCHENZ ET AL: "Functional Analysis of Complex Hepatitis B Virus Variants Associated With Development of Liver Cirrhosis" GASTROENTEROLOGY, ELSEVIER, PHILADELPHIA, PA, Bd. 131, Nr. 3, 1. September 2006 (2006-09-01), Seiten 765-780, XP005659135 ISSN: 0016-5085
- NIESTERS H.G.M. ET AL.: 'The added value of molecular analysis in the source and contact tracing ofRT hepatitis B transmission in a community', [Online] 05 April 2006, Hepatitis B virus isolate s02003094 S protein gene, complete cds. Gefunden im Internet: <URL:http://ibis/IBIS/exam/dbfetch.jsp?id=E M_VI:DQ412279> [gefunden am 2011-06-20]
- LAZAREVIC I ET AL: "Distribution of HBV genotypes, subgenotypes and HBsAg subtypes among chronically infected patients in Serbia", DISTRIBUTION OF HBV GENOTYPES, SUBGENOTYPES AND HBSAG SUBTYPES AMONG CHRONICALLY INFECTED PATIENTS IN SERBIA, vol. 152, no. 11, 6 August 2007 (2007-08-06), pages 2017-2025, XP019565409, ISSN: 1432-8798, DOI: DOI:10.1007/S00705-007-1031-0

## Beschreibung

Die Erfindung betrifft Sequenzen einer neuen Mutante oder Variante des Hepatitis B surface Antigens (HBsAg) und Methoden, diese Genom- und Protein-Variante sowie dagegen gerichtete Antikörper aus Patientenproben zu detektieren.

Die neuen Sequenzen führen zu 7, in dieser Kombination noch nicht bekannten Aminosäure-Austauschen (Substitutionen) in dem Hepatitis B surface Antigen, HBsAg im Bereich der Aminosäurepositionen 100 bis 180 der Aminosäuresequenz des surface Antigens, wobei sich sechs Substitutionen in der Region der a-Determinante befinden (aa 101 bis aa 180) sowie eine Substitution in unmittelbarer Nachbarschaft davon (aa 100).

Die Erfindung betrifft auch immunchemische Nachweisverfahren zum gleichzeitigen Nachweis dieser neuen HBV-Variante zusammen mit bekannten Varianten/Subtypen sowie die Verwendung der neuen Sequenzen in Verbindung mit bekannten Sequenzen zum gleichzeitigen Nachweis von HBV-spezifischen Antikörpern. Die Antigen bzw. Antikörper-Bestimmungen können jeweils in einem Testansatz differenzierend oder nicht-differenzierend durchgeführt werden.

Schließlich betrifft die Erfindung auch den Nachweis der entsprechenden Nukleinsäuren mit Hilfe sogenannter Nukleinsäure-Tests (z.B. Polymerase Chain Reaction, PCR) mit Hilfe geeigneter Primer sowie die Verwendung der neuen Aminosäuresequenzen zur Herstellung von Impfstoffen.

Das Hepatitis B Virus (HBV) ist bekanntermaßen der Auslöser einer Vielzahl von Erkrankungsverläufen, von milden inapparenten Infektionsverläufen bis hin zu chronisch aktiven und fulminant verlaufenden Leberentzündungen (Virushepatitiden).

Die chronische Infektion mit HBV stellt mit geschätzten 400 Millionen betroffenen Menschen ein globales Gesundheitsproblem dar (Lee, N. Engl. J. Med. 337; 1733-1745 (1997))

Als die am besten geeigneten prophylaktischen Maßnahmen für die weltweit häufig anzutreffende HBV-Infektion gelten die aktive Immunisierung (Stimulation der Antikörperantwort durch Antigengabe) und auch die passive Immunisierung (durch Injektion präformierter Antikörper).

Das HBV gehört zu den Hepadna-Viren und stellt ein Viruspartikel mit einem Durchmesser von 42 nm dar, das aus Kern und Hülle besteht. Das Genom des Virus ist eine partiell doppelsträngige, ringförmige DNA- Sequenz von etwa 3200 Nukleotiden, die mindestens sechs verschiedene virale Gene kodieren (Tiollais et al., Nature 317: 489-495 (1985)). Es liegen vier offene Leserahmen für die Bildung der viralen Proteine vor.

Im S-Gen liegt die Information für das HBV Oberflächen- (surface) Antigen (HBsAg), das auch small protein (S) genannt wird. Daneben gibt es größere Formen, die als large protein (L) und middle protein (M) bezeichnet werden. Allen drei Proteinen gemeinsam ist die 226 Aminosäuren umfassende S-HBsAg Sequenz (Gerlich et al., Viral Hepatitis and Liver Disease, Hollinger et al., William-Wilkens, Baltimore, MD, pages 121-134 (1991)).

Die Proteinregionen vor dem small HBs werden auch Pre-Sl und Pre-S2 bezeichnet. Die Pre-S1-Domäne umfasst, abhängig vom Genotyp, 108 bzw: 119 Aminosäuren, während die Pre-S2-Domäne aus 55 Aminosäuren besteht. Beide Domänen sind in dem L-Protein (389 oder 400 Aminosäuren, je nach Genotyp) enthalten, während das M-Protein nur das Pre-S2 zusammen mit dem S-Antigen umfaßt (281 Aminosäuren). Die Pre-S Proteine weisen unterschiedliche Glykosilierungsgrade auf und tragen die Rezeptoren für die Erkennung der Leberzellen.

Das C-Gen trägt die Information für das Nukleokapsid-Protein, Hepatitis B Core-Antigen (HBcAg). Die Translation dieses Proteins kann bereits in der Pre-C-region starten und zur Bildung von Hepatitis Be-Antigen (HBeAg) führen. Das HBeAg weist gegenüber HBcAg eine andere Faltung und Immunogenität auf. HBeAg kommt im Gegensatz zu HBcAg frei im Serum vor und wird bei positivem Nachweis als Indikator für die Bildung von HBcAg und damit für die Bildung infektiöser Viruspartikel angesehen.

Die im Viruspartikel enthaltene Reverse Transkriptions DNA-Polymerase wird vom P-Gen codiert und für das Transaktivator X-Gen wird eine ursächliche Rolle bei der Entstehung von HBV-assoziierten primären Leberzell-Karzinomen diskutiert.

Der virale Replikationszyklus von HBV umfaßt eine intrazelluläre prä-genomische RNA, die im viralen Nukleocapsid in die in DNA umgeschrieben wird. Da die HBV-eigene Reverse Transkriptase DNA- Polymerase über keine Richtigkeits-Lesefähigkeit (proof-reading capability) verfügt, werden mit relativ hoher Häufigkeit falsche Nukleotide eingebaut. Als Folge weist das HBV eine Mutationsrate auf, die mit ca. 1 Nukleotid/10000 Basen/Infektionsjahr etwa dem 10-fachen dessen entspricht, was andere DNA Viren aufweisen (Blum, Digestion 56: 85-95 (1995); Okamoto et al., Jpn. J. Exp. Med. 57: 231-236 (1987)). Daneben treten auch recht häufig Deletionen und Insertionen auf (Carman et al., Lancet 341: 349-353 (1993)).

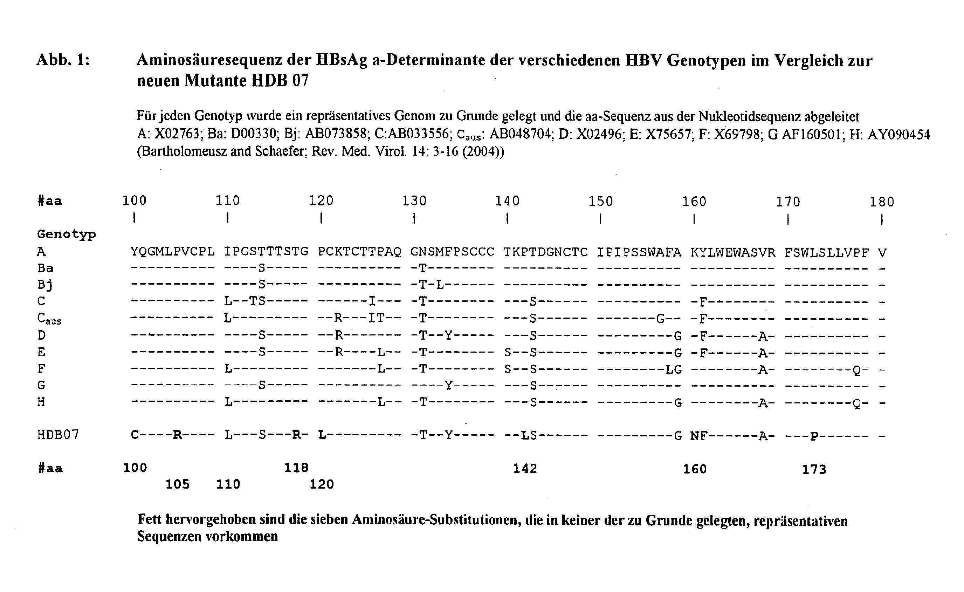

Die resultierende Variabilität von HBV drückt sich unter anderem in dem Auftreten von 9 serologisch definierten Subtypen (Courouce et al., Bibliotheca Haematologica 42: 1 (1976)) und insgesamt mindestens 8 verschiedenen Genotypen aus, die mit A bis H bezeichnet werden (Abb. 1) und eine geographische Verteilung aufweisen. (Norder et al., J. Gen. Virol. 73: 3141-3145 (1992), Norder et al., Virology 198: 489-503 (1994), Norder et al.; Intervirology 47:289-309 (2004)). Außerdem werden eine Reihe von Mutanten beschrieben, bei denen eine Aminosäure oder mehrere ausgetauscht vorliegen, fehlen oder überzählig sind.

Neben natürlicherweise stattfindenden Mutationen (Cooreman et al., Hepatology 30: 1287-1292 (1999) kann eine Gabe von HBV Immunglobulinen und / oder eine antivirale Therapie (z.B. mit Lamivudine) einen Selektionsdruck ausüben, was zum vermehrten Auftreten sogenannter "Escape-Mutanten" führen und die Auftretens-Wahrscheinlichkeit von HBV-Mutanten deutlich erhöhen kann (Terrault et al., Hepatology 28: 555-561 (1998); Tillmann et al., Hepatology 30: 244-256 (1999); Hunt et al., Hepatology 31: 1037-1044 (2000).

Nicht alle HBV-Mutationen führen zu replikationsfähigen Viren und oft liegt eine Koexistenz mit replikationsfahigem Virus vor, was auch die Sequenzierungsgenauigkeit von isolierter DNA limitiert oder gar zur Nichterkennung von veränderten Sequenzen durch PCR, Klonierungsarbeiten mit anschließender Sequenzierung führt, wenn diese quantitativ < 10 % der Gesamt-DNA ausmachen (Cooreman et al., J. Biomed. Sci. 8: 237-247 (2001).

Demnach ist die Isolierung von Mutanten vorteilhaft, wobei die sich anschließende Identifizierung und Charakterisierung einzelner Mutanten möglicherweise zu verbesserten Vakzinen und/oder Diagnostika führt.

Die Immunantwort nach einer Infektion mit HBV ist hauptsächlich gegen die sogenannte a-Determinante als eine allen Hepatitis B Viren gemeinsame Region des S-Proteins gerichtet, die sich auf der Oberfläche der Viruspartikel befindet (Gerlich et al., supra) und die den heterogensten Teil der B-Zellepitope des S-Gens darstellen.

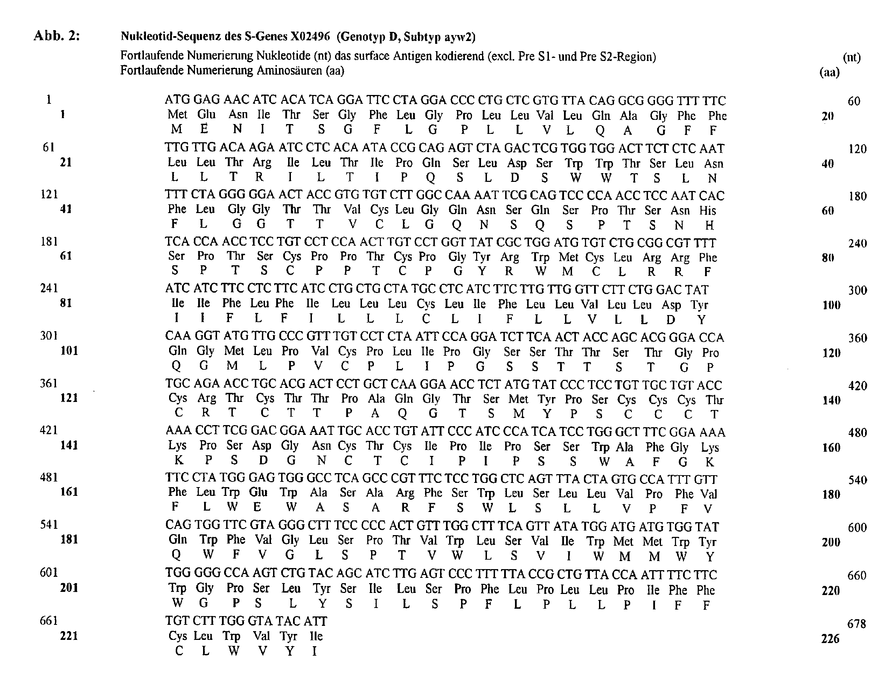

Als Bindestellen für Antikörper werden nach heutigem Kenntnisstand insgesamt mindestens 5 sich teilweise überlappende Epitope auf der a-Determinante zwischen Aminosäure-Position 101 und 180 angenommen (Abb. 1 und 2) wie durch die Anwendung von monoklonalen Antikörpern gezeigt werden konnte (Peterson et al., J. Immunol. 132: 920-927 (1984)). Es handelt sich hauptsächlich um komplexe Konformations-Epitope, die durch mehrere DisulfidBrücken stabilisiert werden. Teilweise liegen auch Sequenz-Epitope vor, die mit Hilfe synthetisch hergestellter zyklischer Peptid-Strukturen dargestellt werden können.

Sogenannte "schützende Antikörper", die nach einer natürlichen Infektion mit HBV im Serum zirkulieren, sind zu 99% gegen die sehr immunogene a-Determinante des HBV gerichtet (Jilg, Vaccine 16: 65-68 (1998). Auf diese Tatsache stützt sich die breite Anwendung der Immunisierung mit Vakzinen, die entweder aus Humanserum isoliert oder gentechnologisch hergestellt wurden und die Verabreichung von Hepatitis B Immunglobulinen, die humane HBV - spezifische Antikörper enthalten. Beide prophylaktischen Strategien beruhen auf dem neutralisierenden Effekt, die HBs-spezifische Antikörper nach Bindung an die "a-Loop-Epitope" entfalten (Carman et al., Hepatology 24: 489-493 (1996), Muller et al., J. Hepatol. 13: 90-96 (1991) und Samuel et al., N. Engl. J. Med. 329: 1842-1847 (1993)).

Ähnlich beruhen die heute weit verbreiteten Diagnostika auf der Bindung von a-Determinantenspezifischen Antikörpern mit Epitopen der a-Determinante. So wird bei der im Blutspendewesen weltweit angewendeten HBsAg-Bestimmung mit immunchemischen Bestimmungsmethoden im Serum von Spendern zirkulierendes HBV Oberflächenantigen mit Antikörpern gegen die a-Determinante (polyklonal oder monoklonalen Ursprungs) nachgewiesen und bei positivem Resultat die entsprechende Blutspende verworfen, um iatrogene HBV Infektionen durch HBV-kontaminiertes Blut zu verhindern. Eine weitere Anwendung der HBsAg-Bestimmung liegt im Nachweis einer vorliegenden akuten HBV-Infektion.

Umgekehrt wird mit der Bestimmung von HBs-spezifischen Antikörpern im Blut von Probanden mit einem positiven Bestimmungsresultat von HBsAg-spezifischen Antikörpern (Anti-HBs) nachgewiesen, daß entweder eine natürliche Infektion abgelaufen oder daß eine durchgeführte Vakzinierung erfolgreich verlaufen ist.

Schließlich beruht auch die Nukleinsäure-Testung z.B. mit Hilfe der Polymerase-Chain-Rektion (PCR, Polymerase-Ketten-Reaktion) auf der Verwendung von Primern (Starter), die für die HBV Nukleotide spezifisch sind.

Auf Grund der zentralen Rolle, die die a-Determinante bei der aktiven Immunisierung (Vakzinierung mit HBV Antigen), der passiven Immunisierung (Schutz durch HBV -spezische Immunglobuline), dem Nachweis von Impferfolg bzw. stattgefundener HBV Infektion (beides mittels Bestimmung von HBsAg-spezischen Antikörpern, Anti-HBs) und schließlich der Sicherheit im Blutspendewesen (HBsAg-Bestimmung und PCR) hat, ist verständlich, daß in Fachkreisen das Auftreten von Mutanten und auch neuen Varianten, insbesondere im Bereich der a-Determinante, mit großer Aufmerksamkeit verfolgt wird.

Es könnten in der a-Determinante des HBV veränderte aber replikationsfähige neue Mutanten und/oder Varianten sowohl das prophylaktische als auch das diagnostische Konzept unterlaufen (Brind et al., J. Hepatol. 26: 228-235 (1997), Fischer et al., Transplant Proc. 31: 492-493 (1999), Ghany et al., Hepatology 27: 213-222 (1998), Protzer-Knolle et al., Hepatology 27: 254-263 (1998), Carman et al., Gastroenterology 102: 711-719 (1992) und Coleman et al., WO 02/079217 Al, (2002)). WO 03/087351 beschreibt Hepatitis B Virus Varianten mit verminderter Reaktivität gegenüber HBsAg-spezifischen Antikörpern. WO 2004/113370 offenbart Sequenzen einer HBsAg Variante sowie Methoden zu deren Nachweis in Patientenproben.

Die Abgrenzung von Varianten und Mutanten des HBV ist nicht scharf, wobei ein diesbezüglicher Vorschlag breite Anwendung findet (Carman, J. Viral Hepat. 4 (suppl.1): 11-20 (1997). Demzufolge sollte die Bezeichnung "Variante" für natürlicherweise vorkommende Subtypen angewendet werden, die ohne bekannte Interferenz, beispielsweise Selektionsdruck durch antivirale Therapie und / oder Immunglobulin-Gabe, auftreten und ein geographisches Verteilungsmuster aufweisen.

Die Charakterisierung und anschließende Klassifizierung der Subtypen erfolgt mit Hilfe monoklonaler Antikörper und basiert auf einem veränderten Reaktionsmuster auf Grund des Austausches von einer oder wenigen Aminosäure(n). Die Grundlage der Klassifizierung stellen die Aminosäurepositionen 122 oder 160 der verbreitetsten HBV Sequenz dar: aa 122 und aa 160 =Lysin, K.

Alle Serotypen enthalten die Gruppen-spezifische a-Determinante, während die aa 122 und zusätzlich 133 und 134 den d- bzw. y-Subtyp und aa 160 die Zugehörigkeit zum w- bzw. r-Subtyp bestimmen. Auf dieser Basis lassen sich HBV Subtypen grob in adr, adw, ayr oder ayw einteilen, die sich weiter in mindestens 9 Sub-Subtypen unterscheiden lassen: ayw1, ayw2, ayw3, ayw4, ayr, adwr2, adw4, adrq+ und adrq- (Swenson et al., J. Virol. Meth. 33: 27-28 (1991), Blitz et al. J. Clin. Microbiol. 36: 648-651, Ashton-Rickardt et al., J. Med. Virol. 29: 204-214 (1989)).

Da diese Klassifizierung eine serologische Reaktivität zur Grundlage hat, muß nicht jede Typisierung notwendigerweise eine Variabilität auf der Aminosäure-Ebene bedeuten, weshalb dem Genotyping auf der S-Gen-Ebene der Vorzug gegeben wird (Ohba et al., Virus Res. 39: 25-34 (1995)).

Subtypen treten aus noch nicht bekannten Gründen in bestimmten geographischen und ethnischen Mustern auf.

Die Bezeichnung Mutation sollte nach Carman solchen Varianten vorbehalten bleiben, die ausschließlich unter Selektionsdruck wie Vakzinierung oder antivirale Therapie entstehen. Es sind bereits viele Mutationen beschrieben worden, von denen manche zu diagnostisch falschen Befundungen führten ( Carman et al., Lancet 345: 1406-1407) und von denen die nachstehend genannten aa-Austausche beispielhaft zitiert werden:

| Consensus: | aa-Position | Mutante: | |
|---|---|---|---|
| I | 110 | V | |
| P | 111 | T | |
| T | 114 | S | |
| T | 116 | S | |
| P | 120 | T / S | |
| T | 123 | A / N | |
| I/T | 126 | A / S | |
| Q | 129 | H / R | |
| K/M | 133 | L | |
| T | 143 | M / L | |
| D | 144 | H / A / E | |
| G | 145 | R / A | |
| A | 157 | R | sowie |
| Cystein Austausche in den aa-Positionen 107, 124, 137, 147 & 149. | | | |

(Coleman, supra; Okamoto et al., Pediatr. Res. 32: 264-268 (1992); Zhang et al., Scand. J. Infect. Dis. 28: 9-15 (1996); Zuckermann et al., Lancet 343: 737-738 (1994)).

Überraschend wurde in einer Probe (Serum/Plasma) eines an Leberentzündung erkrankten Patienten aus Berlin (interne Identifikationsnummer: 126734 / 305024817) ein atypisches Reaktionsmuster von Hepatitis-Markern gefunden.

Neben dem Krankheitsbild deutet auch nachgewiesene Hepatitis B-Virus-DNA auf eine HBV-Infektion hin, ohne daß allerdings der HBsAg-Nachweis mit einem zugelassenen leistungsstarken HBsAg-ELISA gelang.

Eine durchgeführte Sequenzierung führte völlig überraschend zu der in Abb. 3 und 4 dargestellten Nukleotid-Sequenz und der in Abb. 5 und 6 abgebildeten Aminosäure-Sequenz, die beide unerwartet zu dem beschriebenen Substitutionsmuster führten.

Aus diesen Sequenzen wird deutlich, daß es sich völlig überraschend nicht um eine Punktmutation, d.h. Austausch weniger Nukleotide handelt, da insgesamt n=7 Aminosäuren in der Region von aa 100 bis 181 gegenüber den Aminosäuresequenzen der repräsentativen Genotypen (Abb. 1) substituiert vorliegen. Im Hinblick auf die Häufigkeit der Aminosäure-Substitutionen ist überraschend davon auszugehen, daß es sich um eine neue Mutante handelt oder daß die Mutationen so ausgeprägt sind, daß die Konsequenz eher als neue Variante zu beschreiben ist, die im Folgenden als HDB 07-Variante bezeichnet wird.

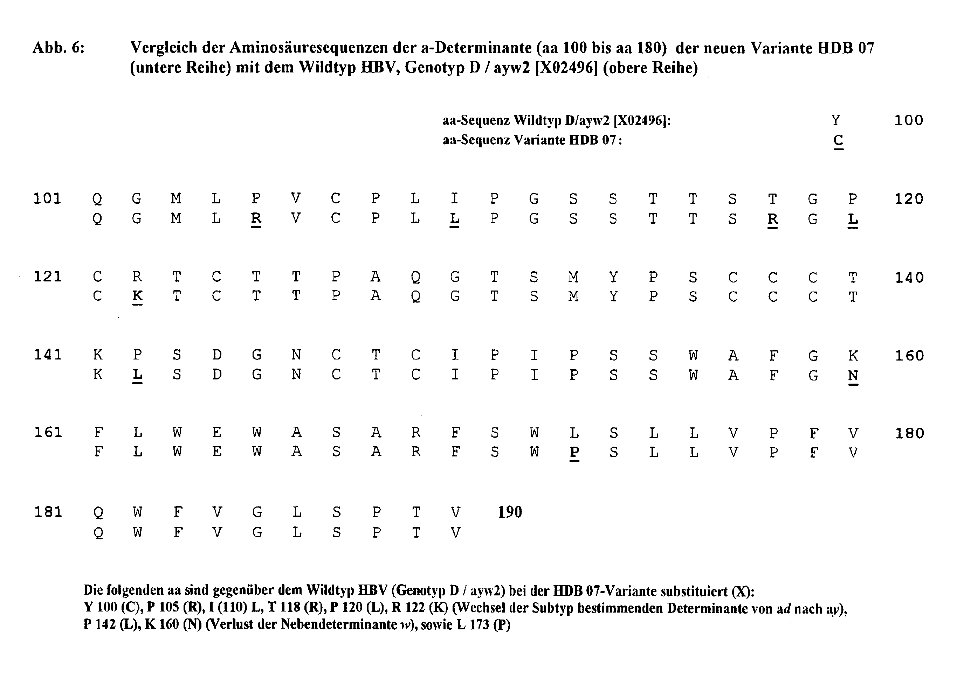

Die Analyse der besten Überstimmung der Aminosäuresequenz der a-Determinante mit bekannten Sequenzen verweist auf Genotyp D (Abb. 1), Subtyp ayw2 (Abb. 2) von dem sich die neue Variante überraschend allerdings in 8 aa-Positionen unterscheidet (Abb. 6). Prominentestes Merkmal sind die Substitutionen an den Positionen 122 und 160. Obwohl die beste Übereinstimmung mit Genotyp D, Subtyp ayw2 erzielt wird, ist die den Subtyp bestimmende Aminosäure an Position 122 überraschenderweise in Bezug auf Genotyp D, Subtyp ayw2 substituiert (Substitution von K anstelle von R), so daß der Subtyp der HDB-07 Variante korrekterweise als ad zu bezeichnen ist. Alle Vergleichssequenzen vom Subtyp ad würden jedoch zu einer schlechteren Gesamtübereinstimmung führen als Genotyp D, Subtyp ayw2. Vollkommen überraschend wurde an Position 160 eine Substitution von N anstelle von K gefunden. Da Position 160 über die Zugehörigkeit zum w- (K an Position 160) bzw. r-Subtyp (R an Position 160) entscheidet, verliert die neue Variante HDB-07 durch diese Substitution diese Subtyp-Zugehörigkeit. Der Verlust der Zugehörigkeit zu einem Subtyp wurde zwar als sehr seltenes Phänomen bereits in der Literatur beschrieben (Okamoto et al., Mol Immunol. 26(2):197-205 (1989)), ein Zusammentreffen eines solchen Phänotyps mit einem substitutionsbedingten Subtypwechsel ist jedoch bislang noch nicht in der Literatur zu finden.

Da bekannt ist, daß Epitope auf der a-Determinante strukturbedingt sind, das heißt als sogenannte Konformationsepitope vorliegen können, ist es wahrscheinlich, daß die Immunogenität und auch die Bindefähigkeit von Antikörpern an die a-Determinante durch den Aminosäure-Austausch in Position # 100 beeinflußt werden kann.

Die vorliegende Erfindung betrifft daher ein Oligo- oder Polypeptid, das die in SEQ ID NO:5 gezeigte Amniosäuresequenz entsprechend den Aminosäurepositionen 100 bis 180 des S-Antigens des Hepatitis B-Virus, welches eine Länge von insgesamt 226 Aminosäure aufweist, umfasst.

Die vorliegende Erfindung betrifft auch ein Oligo- oder Polypeptid umfassend die in SEQ ID NO:3 gezeigte Aminosäuresequenz. Die in SEQ ID NO:3 gezeigte Amniosäuresequenz entspricht dem S-Antigen des Hepatitis B-Virus.

Die Bestimmung der Identität zwischen zwei Aminosäuresequenzen ist dem Fachmann an sich bekannt und kann mit üblichen Computerprogrammen durchgeführt werden. Vorzugsweise wird die Bestimmung der Identität mit dem Computerprogramm "Bestfit" der Genetics Computer Group (Madison, WI) durchgeführt. Die Parameter werden in den Standardeinstellungen (default) verwendet. Vorzugsweise wird die am Prioritätstag der vorliegenden Anmeldung aktuelle Programmversion verwendet. Ein hoher Prozentwert der Identität bedeutet eine hohe Entsprechung, Gleichheit oder Äquivalenz zweier Sequenzen.

Ein Oligo- oder Polypeptid kann auch eine Aminosäuresequenz umfassen, in der gegenüber SEQ ID NO:5 null bis sieben Aminosäuren substituiert, deletiert oder inseriert sind. In der Aminosäuresequenz können auch 0 bis 6, 0 bis 5, 0 bis 4, 0 bis 3, 0 bis 2 Aminosäuren oder 1 Aminosäure gegenüber SEQ ID NO:5 substituiert, deletiert oder inseriert sein. Substitutionen können auch die Aminosäurepositionen betreffen, die den Positionen 100, 105, 110, 118, 120, 142, 116 und 173 des S-Antigens von HBV entsprechen.

Ein Oligo- oder Polypeptid kann auch eine Aminosäuresequenz umfassen, in der gegenüber SEQ ID NO:3 null bis sechs Aminosäuren substituiert, deletiert oder inseriert sind. In der Aminosäuresequenz können auch 0 bis 6, 0 bis 5, 0 bis 4, 0 bis 3, 0 bis 2 Aminosäuren oder 1 Aminosäure gegenüber SEQ ID NO:3 substituiert, deletiert oder inseriert sein.

Das erfindungsgemäße Oligo- oder Polypeptid kann auch eine Aminosäuresequenz umfassen, die eine Teilsequenz von SEQ ID NO:3 mit wenigstens 75 oder wenigstens 80 oder wenigstens 85 oder wenigstens 90 oder wenigstens 95 oder wenigstens 100 aufeinanderfolgenden Aminosäuren von SEQ ID NO:3 ist, wobei diese Teilsequenz wenigstens

alle acht der Positionen 100, 105, 110, 118, 120, 142, 160 und 173 von SEQ ID NO:3 einschließt.

Das erfindungsgemäße Polypeptid kann auch ein Fragment eines HBs-Antigens eines Hepatitis B-Virus umfassen, wobei das Fragment eine Länge von wenigstens 15 Aminosäurren hat, das HBs-Antigen an Position 100 Cystein, an Position 105 Arginin, an Position 110 Leucin, an Position 118 Arginin, an Position 120 Leucin, an Position 142 Leucin, an Position 160 Asparagin und an Position 173 Prolin aufweist, und das Fragment Cystein 100, Arginin 105, Arginin 118, Leucin 120, Leucin 142, Asparagin 160 und/oder Prolin 173 umfaßt. Das Oligo- oder Polypeptid schließt sieben dieser spezifischen Aminosäurereste ein.

Die Gesamtlänge der Oligo- oder Polypeptide ist in der Regel 5 bis 1000 Aminosäuren, vorzugsweise 6 bis 500 Aminosäuren, bevorzugter 7 bis 300 Aminosäuren, am bevorzugtesten 8 bis 200 Aminosäuren. Die Oligo- oder Polypeptide können auch Fremdaminosäuren enthalten, die nicht vom Genom eines Hepatitis B-Virus kodiert werden. So können Aminosäuren enthalten sein, die die Kopplung an feste Phasen erleichtern oder die Kopplung an Markierungssubstanzen ermöglichen. Es können Aminosäuren enthalten sein, die aufgrund der Klonierung entstanden sind und bei der rekombinanten Expression mit exprimiert wurden. Schließlich kann das erfindungsgemäße Oligo- oder Polypeptid ein Fusionsprotein sein, das neben von HBV abgeleiteten Aminosäuren einen Fusionspartner enthält, z. B. eine "tag"-Sequenz, die die Reinigung erleichtert, oder ein Proteinanteil, der die Löslichkeit und/oder Ausbeute bei der rekombinanten Expression erhöht. Derartige Fusionspartner sind dem Fachmann an sich bekannt.

In einer anderen Ausführungsform enthalten die Oligo- oder Polypeptide keine Fremdaminosäuren, die nicht vom Genom eines HBV kodiert werden. Entsprechend bestehen diese Oligo- oder Polypeptide aus einer der oben und/oder in den Ansprüchen beschriebenen Aminosäuresequenzen.

Das erfindungsgemäße Oligo- oder Polypeptid ist vorzugsweiseimmunogen, d.h. es kann eine Antikörperantwort in einem Säugerorgänismus induzieren. Üblicherweise enthält das Oligo- oder Polypeptid wenigstens eine antigene Determinante oder wenigstens ein Epitop. In einer besonderen Ausführungsform enthält das Oligo- oder Polypeptid ein Epitop, das in anderen HBV-Varianten, z. B. in Subtyp ayw2, nicht enthalten ist.

Vorzugsweise umfasst das Oligo- oder Polypeptid eine der Aminosäuresequenzen SEQ ID NO:3, SEQ ID NO:4 und SEQ ID NO:5.

Ein weiterer Aspekt der Erfindung ist ein immunogenes Peptid oder eine Mischung immunogener Peptide, enthaltend eines oder mehrere der in dieser Anmeldung beschriebenen Oligo- oder Polypeptide. Das immunogene Peptid oder die immunogene Mischung kann das/die Oligo- oder Polypeptide alleine oder in Verbindung mit bekannten HBV-Immunogenen enthalten.

Gegenstand der vorliegenden Erfindung sind auch Nukleinsäuremoleküle, die vom Genom der neuen HBV-Variante HDB 07 oder Mutanten davon abgeleitet sind, insbesondere Nukleinsäuremoleküle, die von dem Gen abgeleitet sind, das HBsAg kodiert.

Die Erfindung betrifft daher beispielsweise ein Oligo- oder Polynukleotid, das die Nukleotidsequenz SEQ ID NO:2 umfasst. Die Nukleotidsequenz SEQ ID NO:2 kodiert für die Aminosäuresequenz SEQ ID NO:5.

Gegenstand der Erfindung ist auch ein Oligo- oder Polynukleotid umfassend die Nukleotidsequenz SEQ ID NO:1. Die Nukleotidsequenz SEQ ID NO:1 kodiert für die Aminosäuresequenz SEQ ID NO:3.

Identität ist hier definiert als Grad der Gleichheit zwischen zwei Strängen von zwei DNA-Segmenten. Ausgedrückt wird die Identität als prozentualer Wert, indem die Anzahl identischer Basen zweier zu vergleichender Sequenzen geteilt durch die Länge der kürzeren Sequenz und mit 100 multipliziert wird (Smith et al., Adv. Appl. Mathem. 2: 482-489 (1981).

Die Bestimmung der Identität zwischen zwei Aminosäuresequenzen ist dem Fachmann an sich bekannt und kann mit üblichen Computerprogrammen durchgeführt werden. Vorzugsweise wird die Bestimmung der Identität mit dem Computerprogramm "Bestfit" der Genetics Computer Group (Madison, WI) durchgeführt. Die Parameter werden in den Standardeinstellungen (default) verwendet. Vorzugsweise wird die am Prioritätstag der vorliegenden Anmeldung aktuelle Programmversion verwendet. Ein hoher Prozentwert der Identität bedeutet eine hohe Entsprechung, Gleichheit oder Äquivalenz zweier Sequenzen.

Diese Bewertung ist auch auf Aminosäuresequenzen von Peptiden und Proteinen anwendbar (Dayhoff, Atlas of Protein Sequences and Structure, M.O Dayhoff ed. 5 Suppl. 3: 353-358, Nat. Biom. Res. Found., Washington D.C., USA, Gribskov, Nucl. Acids Res. 14 (6): 6745-66763 (1986).

Ein Oligo- oder Polynukleotid kann eine Nukleotidsequenz umfassen, in der im Vergleich zu SEQ ID NO:2 null bis 10 Nukleotide substituiert, deletiert oder hinzugefügt sind. In der Nukleotidsequenz können auch 0 bis 9, 0 bis 8, 0 bis 7, 0 bis 6, 0 bis 5, 0 bis 4, 0 bis 3, 0 bis 2 Nukleotide oder 1 Nukleotid gegenüber SEQ ID NO:2 substituiert, deletiert oder inseriert sein.

Ein Oligo- oder Polynukleotid kann auch eine Nukleotidsequenz umfassen, die eine Teilsequenz von SEQ ID NO:1 mit wenigstens 8 aufeinanderfolgenden Nukleotiden von SEQ ID NO:1 ist, wobei die Teilsequenz wenigstens eine der Positionen 298, 299, 300, 310, 311, 312, 313, 314, 315, 328, 329, 330, 343, 344, 345, 352, 353, 354, 358, 359, 360, 364, 365, 366, 424, 425, 426, 478, 479, 480, 517, 518 und 519 von SEQ ID NO:1 einschließt. Die Teilsequenz kann wenigstens 9, bevorzugter wenigstens 10, weiter bevorzugt wenigstens 11, am bevorzugtesten wenigstens 12 aufeinanderfolgende Nukleotide der in SEQ ID NO:1 gezeigten Nukleotidsequenz umfassen. In weiteren Ausführungsformen umfasst die Teilsequenz wenigstens 13, wenigstens 14, wenigstens 15, wenigstens 16, wenigstens 17, wenigstens 18, wenigstens 19, wenigstens 20, wenigstens 21, wenigstens 22, wenigstens 23, wenigstens 24, wenigstens 25, wenigstens 26, wenigstens 27, wenigstens 28, wenigstens 29, wenigstens 30, wenigstens 35, wenigstens 40, wenigstens 45, wenigstens 50, wenigstens 60, wenigstens 70, wenigstens 80, wenigstens 90, wenigstens 100, wenigstens 120, wenigstens 150, wenigstens 175, wenigstens 200, wenigstens 250 oder wenigstens 300 aufeinanderfolgende Nukleotide der in SEQ ID NO:1 gezeigten Nukleotidsequenz.

Die Teilsequenz kann zwei, drei, vier, fünf, sechs, sieben, acht, neun, zehn, elf, zwölf, 13, 14, 15, 16, 17, 18, 19, 20, 21, 22, 23, 24, 25, 26, 27, 28, 29 oder alle 30 der Positionen 298, 299, 300, 310, 311, 312, 313, 314, 315, 328, 329, 330, 343, 344, 345, 352, 353, 354, 358, 359, 360, 364, 365, 366, 424, 425, 426, 478, 479, 480, 517, 518 und 519 von SEQ ID NO:1 einschließen.

In einer anderen Ausführungsform umfasst das Oligo- oder Polynukleotid eine Nukleotidsequenz, die unter stringenten Bedingungen, vorzugsweise spezifisch, mit einem zu der Sequenz SEQ ID NO:1 oder zu der SEQ ID NO:2 komplementären Polynukleotid hybridisiert.

Verfahren zur Bestimmung, ob ein gegebenes Oligo- oder Polynukleotid mit einem anderen Polynukleotid hybridisiert, sind dem Fachmann an sich bekannt. Ein spezielles Beispiel für "stringente Bedingungen" sind folgende Bedingungen: a) 16-stündige Inkubation bei 42 °C in einer Lösung enthaltend 50 % Formamid, 5xSSC (150 mM NaCl, 15 mM Trinatriumcitrat), 50 mM Natriumphosphat pH 7,6, 5 x Denhardt's Lösung, 10 % Dextransulfat und 20 µg/ml denaturierte, gescherte Lachssperma-DNA; b) anschließend Waschen in 0,1 x SSC bei ungefähr 65 °C. Hybridisierungs- und Waschbedingungen sind dem Fachmann an sich bekannt und beispielhaft in Sambrook et al., Molecular Cloning: A Laboratory Manual, Second Edition, Cold Spring Harbor, N.Y., (1989) angegeben. Eine Nukleotidsequenz hybridisiert spezifisch an ein gegebenes Polynukleotid, wenn sie nicht oder wesentlich schwächer an andere Nukleotidsequenzen hybridisiert. Im vorliegenden Fall kann das bedeuten, dass die Nukleotidsequenz nicht oder nur schwach an für HBsAg kodierende Polynukleotide aus herkömmlichen HBV-Varianten (z. B. Genotyp D, Subtyp ayw2) hybridisiert.

Die Erfindung betrifft auch ein Oligo- oder Polynukleotid umfassend eine Nukleotidsequenz, die für ein erfindungsgemäßes Oligo- oder Polypeptid kodiert, wie es in dieser Anmeldung beschrieben ist. Ein weiterer Aspekt der Erfindung ist ein Oligo- oder Polynukleotid umfassend eine zu den oben beschriebenen Nukleotidsequenzen komplementäre Nukleotidsequenz.

Die Mindestlänge der Oligo- oder Polynukleotide beträgt 6, vorzugsweise 8, bevorzugter 10, am bevorzugtesten 12 Nukleotide. Die Gesamtlänge des Oligo- oder Polynukleotids ist in der Regel 6 bis 3000 Nukleotide, vorzugsweise 6 bis 1500 Nukleotide, bevorzugter 8 bis 900 Nukleotide, am bevorzugtesten 8 bis 600 Nukleotide. Die Oligo- oder Polynukleotide können auch Nukleotide enthalten, die nicht vom Genom eines Hepatitis B-Virus herstammen. So können Nukleotide enthalten sein, die für bestimmte Aminosäuren kodieren, die gewünschte Funktionen erfüllen sollen, wie oben beschrieben. Es können Nukleotide enthalten sein, die aufgrund der Klonierung entstanden sind, z. B. um bestimmte Schnittstellen einzuführen. Schließlich kann das erfindungsgemäße Oligo- oder Polynukleotid für ein Fusionsprotein kodieren, das neben von HBV abgeleiteten Aminosäuren einen Fusionspartner enthält, z. B. eine "tag"-Sequenz, die die Reinigung erleichtert, oder ein Proteinanteil, der die Löslichkeit und/oder Ausbeute bei der rekombinanten Expression erhöht. Derartige Fusionspartner und die sie kodierende DNA sind dem Fachmann an sich bekannt.

Bevorzugte Oligo- oder Polynukleotide der vorliegenden Erfindung umfassen eine Nukleotidsequenz, die ausgewählt ist aus der Gruppe bestehend aus SEQ ID NO:1 und SEQ ID NO:2.

Die erfindungsgemäßen Polynukleotide können auch markiert sein, beispielsweise durch eine Fluoreszenzmarkierung oder eine radioaktive Markierung. Derartige Polynukleotide können vorteilhaft in einer Hybridisierungsreaktion oder einer Polymerasekettenreaktion (PCR) eingesetzt werden.

Die Erfindung betrifft auch einen Vektor oder ein Plasmid enthaltend ein Oligo- oder Polynukleotid gemäß vorliegender Erfindung. Das Plasmid kann beispielsweise ein Klonierungsvektor sein, der dazu dient, die Nukleinsäure in Wirtszellen zu vermehren oder bestimmte Restriktionsschnittstellen zur Verfügung zu stellen. Expressionsvektoren sind Vektoren, die die Expression der klonierten Nukleinsäure in Wirtszellen erlauben. Wirtszellen können verschiedene prokaryontische oder eukaryontische Zellen sein. Prokaryontische Wirtszellen sind z. B. bakterielle Zellen wie *E. coli*-Zellen. Die erfindungsgemäßen Expressionsvektoren können bestimmte Steuerelemente wie z.B. Promotoren oder Bindungsstellen für Repressionsfaktoren enthalten. In einer weiteren Ausführungsform enthalten die Expressionsvektoren einen Nukleinsäureabschnitt, der für einen Teil eines Fusionsproteins kodiert.

Die Erfindung betrifft ebenfalls eine Zelle, z. B. eine Wirtszelle, die ein erfindungsgemäßes Polynukleotid, Plasmid oder einen erfindungsgemäßen Vektor enthält. Die Wirtszellen können unter geeigneten Bedingungen kultiviert werden, so dass eine Transkription der enthaltenen Nukleinsäure und nachfolgende Translation stattfindet. Die Erfindung betrifft auch ein Verfahren zur Herstellung eines Polypeptids, bei dem man ein Polynukleotid, ein Plasmid oder einen Expressionsvektor der Erfindung in Wirtszellen einbringt und die Wirtszellen unter Bedingungen kultiviert, die zur Expression des Polypeptids führen. Gegebenenfalls kann anschließend das Polypeptid aus den Wirtszellen gewonnen werden. Vorzugsweise findet die Herstellung des Polypeptids in Bakterien, am bevorzugtesten in *E. coli*-Zellen statt. Geeignete Mittel und Bedingungen zur Kultivierung sind beispielsweise in Ausubel et al. (1993) "Current Protocols in Molecular Biology" beschrieben. Die Gewinnung des exprimierten Polypeptids geschieht nach für den Fachmann an sich bekannten Methoden. Verschiedene Verfahren zur Proteinreinigung sind beispielsweise beschrieben in Scopes R. (1994) "Protein Purification: Principles and Practice" (3rd edition) Springer Verlag.

Die Polypeptide und Peptide der vorliegenden Erfindung können jedoch auch chemisch durch bekannte Verfahren wie z.B. Festphasensynthese hergestellt werden. Ebenso können die erfindungsgemäßen Polynukleotide durch bekannte Verfahren der chemischen Synthese hergestellt werden. Durch chemische Synthese erhaltene Polynukleotidfragmente können dann auch enzymatisch durch Ligasen verknüpft werden. Die erfindungsgemäßen Oligo- oder Polynukleotide können auch durch "site-directed mutagenesis" aus bekannten Sequenzen hergestellt werden, indem an bestimmten Positionen Punktmutationen eingeführt werden. Derartige Verfahren sind dem Fachmann an sich bekannt.

Ein Antikörper, der an ein erfindungsgemäßes Oligo- oder Polypeptid bindet, kann auf bekannte Weise hergestellt werden, entweder mittels eines Oligo- oder Polypeptids der Erfindung oder eines Fragments davon (Harlow und Lane (1988) Antibodies: A Laboratory Manual; Cold Spring Harbor Laboratory). Es kann sich um polyklonale oder monoklonale Antikörper handeln, monoklonale Antikörper sind aber bevorzugt. Vorzugsweise handelt es sich um spezifische Antikörper, die gegen das HBsAg der neuen HBV-Variante gerichtet sind, die aber HBsAg aus anderen HBV-Varianten, z. B. Genotyp D Subtyp ayw2, nicht erkennen. Solche Antikörper können erhalten werden, indem aufgrund eines Sequenzvergleichs der Aminosäuresequenzen des neuen HBsAg und HBsAg aus bekannten Stämmen Peptide ermittelt werden, die spezifisch für das neue HBsAg sind, und diese Peptide zur Herstellung der Antikörper verwendet werden. Es ist auch möglich, eine Mischung polyklonaler Antikörper herzustellen und sie durch Inkubation mit bekanntem HBsAg zu depletieren. In einer anderen Ausführungsform erkennt der Antikörper nicht nur das neue HBsAg, sondern auch bekannte HBsAg-Varianten. Dadurch ist es möglich, gleichzeitig verschiedene Varianten von HBsAg zu detektieren.

Der Antikörper kann an ein Oligo- oder Polypeptid binden, das aus einer Aminosäuresequenz besteht, die ausgewählt ist aus der Gruppe bestehend aus SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 und SEQ ID NO:17. Besonders bevorzugt bindet der Antikörper an ein Oligo- oder Polypeptid, das aus einer Aminosäuresequenz besteht, die ausgewählt ist aus der Gruppe bestehend aus SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16 und SEQ ID NO:17. In einer besonderen Ausführungsform bindet der Antikörper nicht an die a-Determinanten der bekannten HBV-Genotypen A, B, C, D, E und F (siehe Abb. 1). In einer besonderen Ausführungsform bindet der Antikörper nicht an die a-Determinante von HBV Genotyp D, Subtyp ayw2.

Die Antikörper können polyklonalen oder monoklonalen tierischen oder menschlichen Ursprungs sein.

Die Erfindung betrifft auch einen Testkit zum Nachweis von Hepatitis B-Viren, enthaltend ein erfindungsgemäßes Oligo- oder Polypeptid und/oder ein erfindungsgemäßes Oligo- öder Polynukleotid.

Die Erfindung bezieht sich auch auf ein immunogenes Peptid oder eine Mischung immunogener Peptide, enthaltend ein oder mehrere erfindungsgemäßen Oligo- oder Polypeptide alleine oder in Verbindung mit bekannten HBV-Immunogenen.

Ein Verfahren zum Nachweis eines Hepatitis B-Antigens, ist dadurch gekennzeichnet, dass (ä) eine Probe mit einem Antikörper unter Bedingungen inkubiert wird, die die Bildung eines Antigen-Antikörper-Komplexes gestatten; und (b) ein Antigen-Antikörper-Komplex, der den Antikörper enthält, nachgewiesen wird.

Es können monoklonale oder polyklonale Antikörper (oder Mischungen bzw. Fragmente davon oder Mischungen von Fragmenten), die mit Epitopen der neuen HBV Variante reagieren, dazu verwendet werden, die a-Determinante der erfindungsgemäßen HBV Variante in Form der gesamten Polypeptidsequenz oder Teilen davon in Untersuchungsproben zu bestimmen: HBsAg der HDB 07-Variante.

Dem Fachmann ist eine Vielzahl von Bestimmungsmethoden geläufig, bei denen mit einem oder mehreren monoklonalen Antikörper(n) oder polyklonalen Antikörpern (oder Mischungen davon bzw. Fragmenten oder Mischungen von Fragmenten), die spezifisch für die a-Determinante der HBV Variante sind, Immunkomplexe gebildet oder deren Bildung gehemmt werden.

Eine spezielle Ausführung stellt der sogenannte Enzymimmunoassay dar, von dem ein mögliches Testprinzip im folgenden beispielhaft beschrieben wird, ohne jedoch die erfindungsgemäße Idee darauf zu beschränken:
Bei dem sehr weit verbreiteten sogenannten Sandwich-Prinzip werden auf einem geeigneten Träger (z.B. Mikropartikel oder Oberfläche von Vertiefungen einer Mikrotitrationsplatte) immobilisierte Antikörper oder Fragmente davon mit der Untersuchungsprobe inkubiert. An die Antikörper gebundenes HBsAg wird nach Entfernen überschüssiger Probe detektiert, indem eine weitere Inkubation erfolgt mit Anti-HBs-Antikörpern (monoklonal oder polyklonal oder Fragmente bzw. Mischungen dieser Fragmente), die mit einer Sonde versehen sind. Als Sonde wird häufig ein Enzym eingesetzt, dessen katalytische Umsetzung (nach Entfernen des überschüssigen Reagenzes) eines geeigneten Substrates zu einer Farbreaktion führt, die photometrisch gemessen wird und deren Intensität dem in der Probe vorhandenen HBsAg-Gehalt proportional ist.

Neben dieser speziellen Ausführungsform sind auch Methoden bekannt, die homogener Natur sind (d.h. keine bound/free Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. Das Biotin/Streptavidin-System).

All diese Ausführungsformen entsprechen dem Stand der Technik, so dass unter "Bestimmung von HBsAg der neuen HBV Variante" mit der vorliegenden Erfindung alle Verfahren verstanden werden, die sich zum Nachweis von Polypeptidsequenzen oder Antigenen der neuen HBV Variante eignen, ungeachtet ob alleine das HBsAg der neuen Variante bestimmt wird oder in Verbindung mit HBsAg von bekannten a-Determinanten und/oder bekannten Mutationen in der a-Region.

Ebenso ist es möglich, aus ökonomischen Gründen eine HBsAg-Bestimmung mit einer Nachweismethode gegen einen weiteren Analyten (z.B. HIV-Antigen oder die gleichzeitige Bestimmung von HBV Varianten-HBsAg und dagegen gerichtete spezifische Antikörper) in einem Testansatz (differenzierend oder nicht-differenzierend) zu kombinieren.

Die Erfindung betrifft auch ein Verfahren zum Nachweis von Antikörpern, die gegen ein Hepatitis B-Virus-Antigen gerichtet sind, dadurch gekennzeichnet, dass (a) eine Probe mit einem erfindungsgemäßen Oligo- oder Polypeptid unter Bedingungen inkubiert wird, die die Bildung eines Antigen-Antikörper-Komplexes gestatten; und (b) der Antikörper-Antigen-Komplex, der das Oligo- oder Polypeptid enthält, nachgewiesen wird.

Eine spezielle Ausführung stellt der sogenannte Enzymimmunoassay dar, von dem ein mögliches Testprinzip im folgenden beispielhaft beschrieben wird, ohne jedoch die erfindungsgemäße Idee darauf einzuschränken:
Bei dem sehr weit verbreiteten sogenannten Sandwich-Prinzip werden auf einem geeigneten Träger (z.B. Mikropartikel oder Oberfläche von Vertiefungen einer Mikrotitrationsplatte) immobilisierte Epitop-tragende Polypeptid- oder Protein-Sequenzen mit der Untersuchungsprobe inkubiert. An die Epitope gebundene Antikörper werden nach Entfernen überschüssiger Probe detektiert, indem eine weitere Inkubation erfolgt mit Epitop-tragenden Polypeptid- oder Protein-Sequenzen, die mit einer Sonde versehen sind. Als Sonde wird häufig ein Enzym eingesetzt, dessen katalytische Umsetzung (nach Entfernen des überschüssigen Reagenzes) eines geeigneten Substrates zu einer Farbreaktion führt, die photometrisch gemessen wird und deren Intensität dem in der Probe vorhandenen Antikörpergehalt proportional ist.

Neben dieser speziellen Ausführungsform sind auch Methoden bekannt, die homogener Natur sind (d.h. keine bound/free Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. Das Biotin/Streptavidin-System).

Ebenso können die Polypeptidstrukturen der HBV Variante durch anti-idiotypische Antikörper dargestellt werden oder durch Wahl eines geeigneten Testprinzips auch Varianten-spezifische monoklonale oder polyklonale Antikörper zur Bestimmung von Anti-HBs-Antikörpern (z.B. in einem kompetitiven Testformat) herangezogen werden. Es ist ebenso bekannt, dass durch Wahl des Testprinzips auch eine Differenzierung der Immunglobulin-Klassen erfolgen kann (z.B. durch das "indirekte" Verfahren mit einem zweiten Klassen-spezifischen Antikörper (z.B. IgG oder IgM spezifisch) mit Sonde oder mit Hilfe des sogenannten Anti-µ-Prinzips (IgM spezifisch). Natürlich müssen die Methoden und Materialen (inkl. Sonde und Polypeptidsequenzen) dem jeweiligen Ziel angepaßt werden.

All diese Ausführungsformen entsprechen dem Stand der Technik, so dass unter "Bestimmung von Antikörpern, die für die a-Determinante der neuen HDB 07-Variante spezifisch sind" mit der vorliegenden Erfindung alle Verfahren verstanden werden, die sich zum Nachweis von Immunglobulinen und/oder Immunglobulin-Klassen gegen die neue HBV Variante eignen, ungeachtet ob alleine der Antikörper gegen die neue Variante gesucht wird oder in Verbindung mit Antikörpern gegen bekannte a-Determinanten und / oder bekannte Mutationen in der a-Region.

In einem anderen Verfahren kann eine Hepatitis B-Nukleinsäure nachgewiesen werden. Dieses Verfahren ist dadurch gekennzeichnet, dass (a) eine Probe mit einem erfindungsgemäßen Oligo-oder Polynukleotid unter Bedingungen inkubiert wird, die die selektive Hybridisierung des Oligo- oder Polynukleotids mit einer Hepatitis B-Nukleinsäure in der Probe gestatten; und (b) bestimmt wird, ob Polynukleotidduplexe gebildet wurden, die das Oligo- oder Polynukleotid umfassen.

Die Hepatitis B-Nukleinsäure kann auch dadurch nachgewiesen werden, dass (a) eine Probe mit wenigstens einem erfindungsgemäßen Oligo- oder Polynukleotid unter Bedingungen inkubiert wird, die die selektive Hybridisierung des Oligo- oder Polynukleotids mit einer Hepatitis B-Nukleinsäure in der Probe gestatten; (b) eine Polymerasekettenreaktion durchgeführt wird; und (c) bestimmt wird, ob eine Nukleinsäure amplifiziert wurde.

Die Erfindung betrifft auch die Verwendung eines erfindungsgemäßen Oligo- oder Polynukleotids als Primer und/oder als Sonde. Die vorliegenden Nukleotidsequenzen können benutzt werden, um Primer und/oder Gensonden herzustellen, weshalb auch Kits, enthaltend Primer und/oder Sonden zum Nachweis von HBV Varianten-spezifischer Nukleinsäure entweder alleine oder in Verbindung mit bekannten HBV Nukleotidsequenzen in Untersuchungsproben ebenfalls Gegenstand der Erfindung sind.

Auf Basis der vorliegenden Nukleotidsequenzen können Primer entwickelt werden, die in der sog. "Polymerase Chain Reaction" (PCR) Verwendung finden. Die PCR stellt eine Methode dar, um eine gewünschte Nukleotidsequenz einer Nukleinsäure oder eines Nukleinsäuregemisches zu amplifizieren. Dabei werden die Primer jeweils spezifisch durch eine Polymerase mit der gewünschten Nukleinsäure als Leseraster verlängert. Nach Dissoziation von dem Originalstrang werden neue Primer hybridisiert und wiederum durch die Polymerase verlängert. Durch Wiederholung dieser Zyklen wird eine Anreicherung der gesuchten Zielsequenz-Moleküle erreicht.

In Bezug auf Nukleinsäuretests (NAT) ist es möglich, Nukleotid-Sequenzen der vorliegenden Erfindung zu verwenden, um DNA- Oligomere von 6-8 Nukleotiden oder größer herzustellen, die geeignet sind, als Hybridisierungssonden das virale Genom der hier beschriebenen HBV Variante in Personen nachzuweisen, die möglicherweise die Virus-Variante tragen, oder z.B. im Blutspendewesen Blutkonserven auf Vorhandensein des Varianten-Genoms entweder gezielt oder in Kombination mit dem Nachweis von Nukleotidsequenzen bekannter HBV Varianten und/oder HBV-Mutatanten zu screenen.

Ebenso können auf Basis der gefundenen Nukleotidsequenzen der neuen HBV Variante entsprechende Primer entwickelt werden, die für die neue Variante spezifisch sind oder sowohl die neue Variante als auch im Stand der Technik bekannte Varianten detektieren können.

Ein weiterer Gegenstand der vorliegenden Erfindung ist ein isoliertes Hepatitis B-Virus, das ein HBs-Antigen aufweist, das die Aminosäuresequenz SEQ ID NO:5 umfasst. Schließlich umfasst die Erfindung auch Kulturen von Gewebezellen, die mit der erfindungsgemäßen HBV-Variante infiziert sind ebenso wie die isolierte-HBV Variante selbst. Auch eine immunogene Zubereitung, die die attenuierte oder inaktivierte HDB 07-Variante von HBV enthält, ist Gegenstand der Erfindung.

Die Erfindung betrifft auch die Verwendung eines erfindungsgemäßen Oligo- oder Polynukleotids oder eines erfindungsgemäßen Oligo- oder Polypeptids zur Herstellung eines Arzneimittels zur Behandlung oder Vorbeugung einer HBV-Infektion. Insbesondere können die erfindungsgemäßen Oligo- oder Polynukleotide bzw. Oligo- oder Polypeptide zur Herstellung eines Impfstoffs gegen HBV verwendet werden.

Ferner schließt die Erfindung auch eine Vakzine ein umfassend ein Polypeptid der vorliegenden Erfindung und ein gebräuchliches Adjuvans (z.B. Freund'sches adjuvans, Phosphat-gepufferte Saline o.ä.). Eine derartige Vakzine kann dazu verwendet werden, um die Bildung von Antikörpern in Säugetieren anzuregen. Ähnlich umfasst die Erfindung ein Partikel, das eine Nicht-Varianten spezifische Aminosäure-Sequenz beinhaltet, die eine Partikel-Bildung induziert zusammen mit einem Epitop-enthaltenden Polypeptid, das spezifisch für die erfindungsgemäße HBV Variante ist.

Die Nukleotidsequenzen der Erfindung können auch dazu verwendet werden, sog. Antisense Oligonukleotide darzustellen (gegebenenfalls für therapeutische Zwecke).

Weitere Aspekte der vorliegenden Erfindung sind die folgenden Gegenstände (1) bis (15):
(1) Ein isoliertes Oligo- oder Polynukleotid mit einer der Sequenzen ausgewählt aus der Gruppe von SEQ ID NO:1 bis SEQ ID NO:2:
   **SEQ ID NO:1**
   **SEQ ID NO:2** GTT *242*
   **SEQ ID NO:18**
      AGCAGGGGACTA
   **SEQ ID NO:19**
      AGCAGGGGACTATGCAAA
   **SEQ ID NO:20**
      AGCAGGGGACTATGCAAAACCTGC
   **SEQ ID NO:21**
      AGCAGGGGACTATGCAAAACCTGCACGACTCCTGCTCAAGGAACC
   **SEQ ID NO:22**
   **SEQ ID NO:23**
      TTCGGAAACTTC
   **SEQ ID NO:24**
      TGGGCTTTCGGAAACTTC
   **SEQ ID NO:25**
      CCATCATCCTGGGCTTTCGGAAACTTC
   **SEQ ID NO:26**
      AATTGCACCTGTATTCCCATCCCATCATCCTGGGCTTTCGGAAACTTC
   **SEQ ID NO:27**
(2) Ein Oligo- oder Polynukleotid nach (1), das mit einem Oligo- oder Polynukleotid, welches eine zu einer der Sequenzen, ausgewählt aus der Gruppe von SEQ ID NO:1 bis SEQ ID NO:2, komplementär Sequenz hat, unter stringenten Bedingungen hybridisiert.
(3) Ein isoliertes Oligo- oder Polynukleotid, welches für HBs-Antigen des Hepatitis B-Virus **kodiert** und ein Oligo- oder Polynukleotid nach (1) oder (2) enthält.
(4) Ein Fragment eines Oligo- oder Polynukleotids, welches für HBs-Antigen des Hepatitis B-Virus kodiert, dadurch gekennzeichnet, dass das Fragment ein Oligo- oder Polypeptid nach (1) oder, (2) enthält.
(5) Ein isoliertes Oligo- oder Polynukleotid, welches für die a-Determinante des HBs-Antigens des Hepatitis B-Virus kodiert und ein Oligo- oder Polynukleotid nach (1) oder (2) enthält.
(6) Ein Primer, der für ein Oligo- oder Polynukleotid gemäß einem der Gegenstände (1) bis (5) spezifisch ist.
(7) Ein Vektor, der mindestens ein Oligo- oder Polynukleotid gemäß einem der Gegenstände (1) bis (4) enthält.
(8) Eine Wirtszelle, die einen Vektor nach (7) enthält.
(9) Ein Oligo- oder Polypeptid welches durch ein Oligo- oder Polynukleotid nach einem der Gegenstände (1) bis (4) kodiert wird.
(10) Isoliertes Oligo- oder Polypeptid, das eine Aminosäuresequenz ausgewählt aus der Gruppe von SEQ ID NO:3 bis SEQ ID NO:5 hat:
   **SEQ ID NO:3** C L W V Y I ***226***
   **SEQ ID NO:4**
   **SEQ ID NO:5**
   **SEQ ID NO:6**
      S T T S R
   **SEQ ID NO:7**
      T T S R G
   **SEQ ID NO:8**
      T S R G L
   **SEQ ID NO:9**
      S R G L C
   **SEQ ID NO:10**
      R G L C K
   **SEQ ID NO:11**
      G L C K T
   **SEQ ID NO:12**
      L C K T C
   **SEQ ID NO:13**
      W A F G N
   **SEQID NO:14**
      A F G N F
   **SEQ ID NO:15**
      F G N F L
   **SEQ ID NO:16**
      G N F L W
      N F L W E
(11) Isoliertes Polypeptid entsprechend der Sequenz des HBs-Antigens des Hepatitis B-Virus, dadurch gekennzeichnet, dass es ein Oligo- oder Polypeptid gemäß einem der Gegenstände (9) bis (10) enthält.
(12) Fragment eines Polypeptids, welches der Sequenz des HBs-Antigens des Hepatitis B-Virus entspricht, dadurch gekennzeichnet, dass das Fragment ein Oligo- oder Polypeptid gemäß einem der Gegenstände (9) bis (10) enthält.
(13) Isoliertes Polypeptid, welches für die a-Determinante des HBs-Antigens des Hepatitis B-Virus kodiert, dadurch gekennzeichnet, dass es ein Öligo- oder Polypeptid gemäß einem der Gegenstände (9) bis (10) enthält.
(14) Testkit zum Nachweis oder zur Bestimmung mittels einer Hybridisierungsreaktion einer Nukleinsäure, die für eine Variante oder Mutante des Hepatitis B-Virus spezifisch ist, unter Verwendung mindestens eines Oligo- oder Polynukleotids gemäß einem oder mehreren der Gegenstände (1) bis (6).
(15) Testkit zum immunchemischen Nachweis oder zur immunchemischen Bestimmung eines gegen eine Variante oder Mutante des Hepatitis B-Virus gerichteten Antikörpers unter Verwendung mindestens eines Oligö- oder Polypeptids gemäß einem der Gegenstände (9) bis (13).

Zusätzlich beinhaltet die vorliegende Erfindung eine isolierte Nukleotid-Sequenz, die die vorliegende erfindungsgemäße Variante der a-Determinante des Hepatitis B surface Antigens (HBsAg) in den Aminosäuren-Positionen zwischen aa 1 und 226 kodiert bzw. zu einem Peptid-Produkt führt, das in der aa-Sequenz mit der in Abb. 6 dargestellten SEQ. ID NO:4 übereinstimmt

Des weiteren beinhaltet die vorliegende Erfindung einen Vektor, umfassend eine oder mehrere der genannten Nukleotid-Sequenzen wie auch eine Wirtszelle, die diesen Vektor enthält und eine Methode zur Darstellung eines entsprechenden Polypeptids aus der a-Determinante, umfassend die Inkubation der oben genannten Wirtszelle über Zeiten und unter Bedingungen, die für die Expression des Polypeptides erforderlich sind.

Eine isolierte HBV Variante ist ebenfalls Gegenstand der Erfindung, wobei das Virus eine a-Determinante aufweist, die den aa-Sequenzen mindestens zwischen ca. Position 100 und 160 entspricht.

Auch eine immunogene Mischung zur Erzeugung polyklonaler oder monoklonaler Antikörper ist Gegenstand der vorliegenden Erfindung umfassend das beschriebene isolierte HBV oder ein bzw. mehrere der beschriebenen Poypeptide.

Die Erfindung beinhaltet auch eine Polynukleotid-Sonde, enthaltend eine HBV Genom-Sequenz, welche durch Substitution von Aminosäuren zu einer modifizierten a-Determinanten führt, die mit der beschriebenen aa-Sequenz der neuen HBV-Variante identisch ist.

Auch Kits zum Nachweis von Polynukleotiden der HBV-Variante mit Hilfe der genannten Sonde wie auch Kits zum Nachweis von Antikörpern, die für die Variante oder Epitope davon spezifisch sind, sind ebenso Gegenstand der Erfindung, wie die Methoden des Nachweises von Polynukleotiden und Antikörpern, umfassend eine Inkubation zur Bildung entsprechender Komplexe und Nachweis dieser Komplexe durch geeignete dem Fachmann bekannte Verfahren.

Die Ausführungsformen dieser Kits und Nachweismethoden können zum spezifischen und alleinigen Nachweis von Nukleotiden und gegen das Antigen der HBV-Variante gerichteter Antikörper ausgelegt sein oder supplementär, d.h. den zusätzlichen Nachweis der erfindungsgemäßen Varianten-Analyten zu derzeitig bekannten HBV-Nukleotiden, bzw. Antikörpern gestatten.

Analog kann eine immunogene Mischung von erfindungsgemäßen Polypeptidsequenzen auch in Verbindung mit bekannten Antigenen z.B. für die Verbesserung der Wirksamkeit einer Vakzine angewendet werden.

Die vorliegende Erfindung beschreibt eine neue Variante des Hepatitis B Virus (HBV), die eine völlig neue a-Determinante als Resultat von Aminosäure-Austauschen in den nachfolgenden aa-Positionen der S-HBsAg Sequenz aufweist. Für die Beschreibung der Aminosäuren wird der 1-Buchstaben-Code verwendet:

| aa von HDB 07 | aa-Position | aa von ayw2/Genotyp D |
|---|---|---|
| R | 105 | P |
| L | 110 | I |
| R | 118 | T |
| L | 120 | P |
| K | 122 | R |
| L | 142 | P |
| N | 160 | K |
| P | 173 | L |

Außerdem liegt in Position aa 100 von HDB 07 Cystein (C) statt Tyrosin (Y) vor:

| | | |
|---|---|---|
| C. | 100 | Y. |

Diese aa-Substitutionen lassen sich auf entsprechende Nukleotid-Substitutionen der entsprechenden Codons zurückführen.

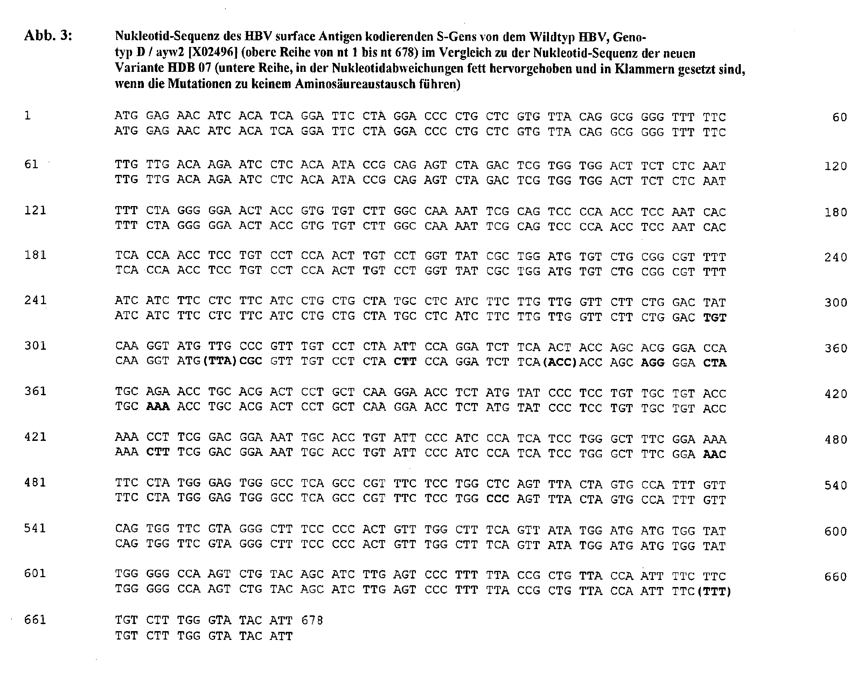

Die vorliegende Erfindung betrifft eine isolierte Nukleotid-Sequenz, die für die a-Determinante des Virus kodiert (Abb. 3 sowie SEQ ID No: 1).

Die Erfindung umfaßt auch Nukleotide mit mindestens 65 % Übereinstimmung, bevorzugt mindestens 75% Übereinstimmung und besonders bevorzugt mit mindestens 90% Übereinstimmung mit der Nukleotid-Sequenz der vorliegenden Erfindung bzw. Fragmenten davon sowie dazu komplementäre Sequenzen.

Die Erfindung umfaßt auch Polypeptide, die von, oben beschriebenen Nukleotid-Sequenzen kodiert werden, insbesondere solche Aminosäuresequenzen, die die a-Determinante des HBsAg bestimmen.

Unter einem Nukleotid-Fragment wird eine konsekutive Folge von mindestens 9, bevorzugt 9-15, besonders bevorzugt 15-21 und sogar ganz besonders bevorzugt 21-60 Nukleotide aus der Nukleotidsequenz der neuen HBV-Variante verstanden, wobei auch Mischungen derartiger Nukleotid-Fragmente naheliegen.

Ein Polypeptid-Fragment wird als Folge von mindestens 3, bevorzugt 3-5, besonders bevorzugt 5-7 und sogar ganz besonders bevorzugt 7-20 Aminosäuren aus der a-Determinante der neuen HBV-Variante verstanden, wobei auch Mischungen derartiger Polypeptid-Fragmente unter diese Erfindung fallen.

Unter die vorliegende Erfindung fällt auch eine isolierte Nukleotid-Sequenz, die hybridisierbar ist und zu Nukleotid-Sequenzen führt, die den Nukleotidsequenzen des HBsAg der neuen HBV Variante oder Teilen der a-Determinante der neuen HBV Variante entsprechen, komplementär dazu sind oder als Subtyp oder Mutation auf HDB 07 zurückzuführen sind.

Dem Fachmann ist bekannt, daß eine Nukleotidsequenz nach deren Isolierung nach Methoden gemäß Stand der Technik in prokaryontische (z.B. E. coli), eukaryontische Wirtszellen (z.B. Chinese Hamster Ovary Cell) oder Hefe (z.B. S. Cerevisiae) mit Hilfe eines Vektors oder Konstruktes eingebracht werden können (mit dem Fachmann bekannten Methoden wie z.B. Transfektion, Transformation oder Elektroporation: Molecular Cloning: A Laboratory Manual, 2nd ed., Vol. 1-3, ed Sambrook et al., Cold Spring Harbor Laboratory Press (1989), wobei transiente oder permanente Kulturen angewendet werden können.

Demzufolge umfaßt die vorliegende Erfindung isolierte Nukleotidsequenzen der a-Determinante der neuen HBV Variante, Polypeptide, die durch diese Nukleotide kodiert werden, Vektoren, die Nucleotidsequenzen der a-Determinante der neuen HBV Variante enthalten wie auch die Wirtszelle, in die ein Vektor gebracht wird. Neben der Darstellung von Polypeptiden mit Hilfe eines Expressions-Systems (rekombinant oder gentechnologisch) ist dem Fachmann bekannt, daß analoge Polypeptid-Strukturen auch vollsynthetisch oder direkt durch Reinigung aus der Virusvariante hergestellt werden.

Es ist möglich, die Polypeptide oder Proteine der neuen HBV Variante zur Erzeugung von monoklonalen und/oder polyklonalen Antikörpern zu verwenden, die immunologisch an Bindestellen (Epitope) der a-Determinante der neuen HBV Variante binden. Die Methoden zur Darstellung von Antikörpern sind dem Fachmann bekannt (z.B. Koehler et al., Nature 256-494 (1975), Mimms et al., Vi. 176: 604-619 (1990).

Ferner ist es möglich, die a-Determinante der erfindungsgemäßen HDB 07-Variante in Form der gesamten Polypeptidsequenz oder Teilen davon für die Bestimmung von gegen die HBV Variante gerichteten Antikörpern (Anti-HBsAg-Antikörpern) zu verwenden (siehe oben).

Dem Fachmann ist eine Vielzahl von Bestimmungsmethoden geläufig, bei denen mit Polypeptiden aus der a-Determinante der HBV Variante und Antikörpern tierischen oder menschlichen Ursprungs Immunkomplexe gebildet oder deren Bildung gehemmt werden.

Eine spezielle Ausführung stellt der sogenannte Enzymimmunoassay dar, von dem ein mögliches Testprinzip im Folgenden beispielhaft beschrieben wird, ohne jedoch die erfindungsgemäße Lehre darauf zu beschränken:
Bei dem sehr weit verbreitetet sogenannten Sandwich-Prinzip werden auf einem geeigneten Träger (z.B. Mikropartikel oder Oberfläche von Vertiefungen einer Mikrotitrationsplatte) immobilisierte Epitop-tragende Polypeptid- oder Protein-Sequenzen mit der Untersuchungsprobe inkubiert. An die Epitope gebundene Antikörper werden nach Entfernen überschüssiger Probe detektiert, indem eine weitere Inkubation mit Epitop-tragenden Polypeptid- oder Protein-Sequenzen erfolgt, die mit einer Sonde versehen sind. Als Sonde wird häufig ein Enzym eingesetzt, dessen katalytische Umsetzung eines geeigneten Substrates - nach Entfernen des überschüssigen Reagenzes - zu einer Farbreaktion führt, die photometrisch gemessen wird und deren Intensität dem in der Probe vorhandenen Antikörpergehalt proportional ist.

Neben dieser speziellen Ausführungsform sind auch Methoden bekannt, die homogener Natur sind (d.h. keine bound/free-Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. das Biotin/Strepavidin-System).

Ebenso können die Polypeptidstrukturen der HBV-Variante HDB 07 durch anti-idiotypische Antikörper dargestellt werden oder durch Wahl eines geeigneten Testprinzips auch Variantenspezifische monoklonale oder polyklonale Antikörper zur Bestimmung von Anti-HBs-Antikörpern (z.B. in einem kompetitiven Testformat) herangezogen werden. Es ist ebenso bekannt, daß durch Wahl des Testprinzips auch eine Differenzierung der Immunglobulin-Klassen erfolgen kann (z.B. durch das "indirekte" Verfahren mit einem zweiten Klassenspezifischen Antikörper (z.B. IgG- oder IgM-spezifisch) mit einer Sonde oder mit Hilfe des sogenannten Anti-p-Prinzips (IgM spezifisch)). Natürlich müssen die Methoden und Materialen, einschließlich Sonde und Polypeptidsequenzen, dem jeweiligen Ziel angepaßt werden.

All diese Ausführungsformen sind dem Fachmann bekannt, so daß unter "Bestimmung von Antikörpern, die für die a-Determinante der neuen HDB 07-Variante spezifisch sind" gemäß vorliegender Erfindung alle Verfahren verstanden werden, die sich zum Nachweis von gegen die neue HBV-Variante HDB 07 gerichteten Immunglobulinen und / oder Immunglobulin-Klassen eignen, ungeachtet der Frage, ob alleine der Antikörper gegen die neue Variante gesucht wird oder in Verbindung mit Antikörpern gegen bekannte a-Determinanten und / oder bekannte Mutationen in der a-Region.

Schließlich ist dem Fachmann ebenso bekannt, monoklonale oder polyklonale Antikörper oder Mischungen davon oder Fragmente solcher Antikörper oder Mischungen davon, die mit Epitopen der neuen HBV-Variante HDB 07 reagieren, dazu zu verwenden, die a-Determinante der erfindungsgemäßen HBV-Variante (HBsAg der HDB 07-Variante) in Form der gesamten Polypeptidsequenz oder Teilen davon in Untersuchungsproben zu bestimmen.

Dem Fachmann ist eine Vielzahl von Bestimmungsmethoden geläufig, bei denen mit einem oder mehreren monoklonalen Antikörper(n) oder polyklonalen Antikörpern (oder Mischungen davon bzw. Fragmenten oder Mischungen von Fragmenten), die spezifisch für die a-Determinante der HBV-Variante sind, Immunkomplexe gebildet oder deren Bildung gehemmt werden.

Eine spezielle Ausführung stellt der sogenannte Enzymimmunoassay dar, von dem ein mögliches Testprinzip im folgenden beispielhaft beschrieben wird, ohne jedoch die erfindungsgemäße Idee darauf zu beschränken:
Bei dem sehr weit verbreitetet sogenannten Sandwich-Prinzip werden auf einem geeigneten Träger (z.B. Mikropartikel oder Oberfläche von Vertiefungen einer Mikrotitrationsplatte) immobilisierte Antikörper oder Fragmente davon mit der Untersuchungsprobe inkubiert. An die Antikörper gebundenes HBsAg wird nach Entfernen überschüssiger Probe detektiert, indem eine weitere Inkubation erfolgt mit Anti-HBs-Antikörpern (monoklonal oder polyklonal oder Fragmente bzw. Mischungen dieser Fragmente), die mit einer Sonde versehen sind. Als Sonde wird häufig ein Enzym eingesetzt, dessen katalytische Umsetzung eines geeigneten Substrates - nach Entfernen des überschüssigen Reagenzes - zu einer Farbreaktion führt, die photometrisch gemessen wird und deren Intensität dem in der Probe vorhandenen Antikörpergehalt proportional ist.

Neben dieser speziellen Ausführungsform sind auch Methoden bekannt, die homogener Natur sind (d.h. keine bound/free Trennung erfordern), die ganz ohne Sonde auskommen (z.B. Agglutinationsverfahren), mit bloßem Auge auswertbar sind (z.B. radiale Immundiffusion) oder sich anderer Sonden (z.B. radioaktive Isotope oder Chemilumineszenz) bzw. mehrerer Sonden bedienen (wie z.B. das Biotin/Strepavidin-System).

All diese Ausführungsformen sind dem Fachmann bekannt, so daß unter "Bestimmung von HBsAg der neuen HBV Variante" mit der vorliegenden Erfindung alle Verfahren verstanden werden, die sich zum Nachweis von Polypeptidsequenzen oder Antigenen der neuen HBV Variante eignen, ungeachtet der Frage, ob alleine das HBsAg der neuen Variante bestimmt wird oder in Verbindung mit HBsAg von bekannten a-Determinanten und / öder bekannten Mutationen in der a-Region.

Ebenso ist dem Fachmann geläufig, aus ökonomischen Gründen eine HBsAg-Bestimmung mit einer Nachweismethode zum Nachweis eines weiteren Analyten (z.B. HIV-Arttigen oder die gleichzeitige Bestimmung von.H.BV Varianten-HBsAg und dagegen gerichtete spezifische Antikörper) in einem Testansatz (differenzierend oder nicht-differenzierend) zu kombinieren.

In Bezug auf Nukleinsäure-Testung (NAT) ist dem Fachmann ebenso bekannt, Nukleotid-Sequenzen gemäß vorliegender Erfindung zu verwenden, um DNA-Oligomere von 6-8 Nukleotiden oder größer herzustellen, die geeignet sind, als Hybridisierungssonden das virale Genom der hier beschriebenen HBV-Variante in Personen nachzuweisen, die möglicherweise die Virus-Variante HDB 07 tragen oder z.B. im Blutspendewesen Blutkonserven auf Vorhandensein des Varianten-Genoms entweder gezielt oder in Kombination mit dem Nachweis von Nukleotidsequenzen bekannter HBV-Varianten und/oder HBV-Mutanten zu screenen. Ebenso können auf Basis der gefundenen Nukleotidsequenzen der neuen HBV Variante entsprechende Primer entwickelt werden.

Ferner schließt die Erfindung auch eine Vakzine ein, umfassend ein Polypeptid gemäß vorliegender Erfindung und ein gebräuchliches Adjuvans (z.B. Freundsches Adjuvans, Phosphat-gepufferte Saline o.ä.). Eine derartige Vakzine kann dazu verwendet werden, um die Bildung von Antikörpern in Säugetieren einschließlich des Menschen anzuregen. Ähnlich umfaßt die Erfindung ein Partikel, das eine nicht-variantenspezifische Aminosäure-Sequenz beinhaltet, die eine Partikel-Formation induziert zusammen mit einem Epitop-enthaltenden Polypeptid, das spezifisch für die erfindungsgemäße HBV-Variante HDB 07 ist.

Schließlich sind auch diagnostische Reagenzien als Kits Gegenstand der Erfindung, die basierend auf den oben beschriebenen Verfahren einen Nachweis von gegen HBV Varianten-spezifischem Antigen (HBsAg) gerichtete Antikörper (Anti-HBs), entweder als singuläre Bestimmungen oder miteinander oder mit anderen bekannten HBV-Antigenen bzw. spezifisch damit reagierenden Antikörpern bzw. auch mit ganz anderen Analyten kombinierbar.

Zusätzlich können die vorliegenden Nukleotidsequenzen benutzt werden, um Primer und/oder Gensonden herzustellen, weshalb auch Kits, enthaltend Primer und/oder Sonden zum Nachweis von HBV Varianten-spezifischer Nukleinsäure entweder alleine oder in Verbindung mit bekannten HBV Nukleotidsequenzen in Untersuchungsproben ebenfalls Gegenstand der Erfindung sind.

Nuleotidsequenzen gemäß vorliegender Erfindung können auch dazu verwendet werden, sog. Antisense-Oligonukleotide darzustellen (ggfs. für therapeutische Zwecke).

Schließlich können auf Basis vorliegender Nukleotidsequenzen auch Primer entwickelt werden, die in der sog. Polymerase Chain Reaction (PCR) Verwendung finden. Die PCR stellt eine Methode dar, um eine gewünschte Nukleotidsequenz einer Nukleinsäure oder eines Nukleinsäuregemisches zu amplifizieren. Dabei werden die Primer jeweils spezifisch durch eine Polymerase mit der gewünschten Nukleinsäure als Leseraster verlängert. Nach Dissoziation von dem Originalstrang werden neue Primer hybridisiert und wiederum durch die Polymerase verlängert. Durch Wiederholung dieser Zyklen wird eine Anreicherung von Molekülen mit der gesuchten Zielsequenz erreicht.

Weiterhin umfaßt die vorliegende Erfindung auch Kulturen von Gewebezellen, die mit der erfindungsgemäßen HBV-Variante infiziert sind, ebenso wie die isolierte HBV-Variante selbst. Auch eine immunogene Aufbereitung, die die attenuierte oder inaktivierte erfindungsgemäße HDB 07 -Variante von HBV enthält, ist Gegenstand der Erfindung.

Die vorliegende Erfindung ist darüber hinaus in den Patentansprüchen beschrieben.

### Beschreibung der Abbildungen:

**Abb. 1** stellt eine Übersicht der Aminosäure- Sequenzen der a-Determinante von 8 beschriebenen Genotypen des HBV im Vergleich zur Variante HDB 07 dar.
In **Abb. 2** sind die Nukleotid- und Aminosäure-Sequenzen des S-Gens des Genotyps D, Subtyp ayw2 von HBV dargestellt.
**Abb. 3** bildet die Nukleotid-Sequenz des HBV surface Antigens für Subtyp ayw2 des Genotyps D von HBV ab im Vergleich zur Nukleotid-Sequenz von HDB 07.

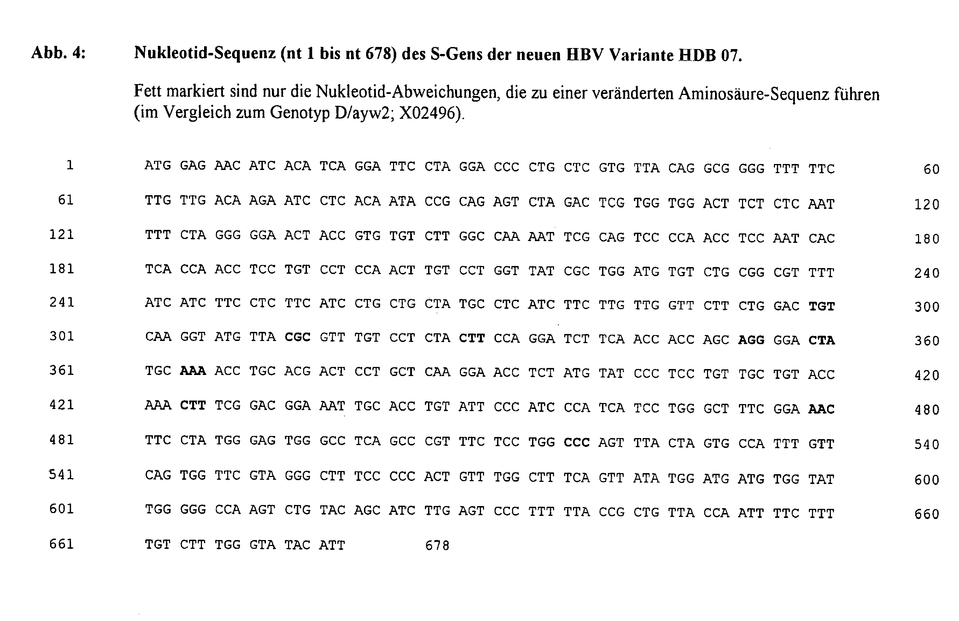

**Abb. 4** fasst die translationsrelevanten Abweichungen der Nukleotid-Sequenz von HDB 07 zusammen.

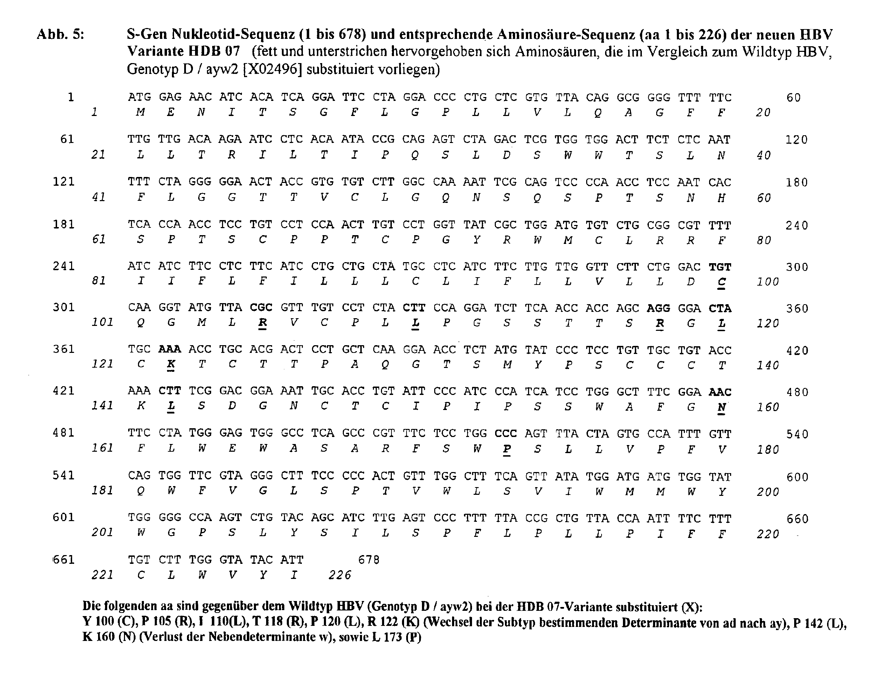

In **Abb. 5** wird die Nukleotid-Sequenz des S-Gens von HDB 07 sowie die entsprechende Aminosäure-Sequenz dargestellt. Die a-Determinante befindet sich zwischen Aminsäure No. 101 und 180 des kleinen HBsAg (Small, S).
**Abb. 6** zeigt die entsprechende Polypeptid-Sequenz der a-Determinante von HDB 07, sowie eng benachbarter Bereiche, die von der in Abb. 5 beschriebenen Nukleotid-Sequenz kodiert werden. Diese werden mit analogen Bereichen des Subtyp ayw2 des Genotyps D von HBV verglichen.

Die nachfolgenden Beispiele erläutern die vorliegende Erfindung näher, ohne daß die Erfindung auf die beschriebenen Beispiele beschränkt ist.

### Beispiel 1: HBsAg-Bestimmung mittels Enzymimmunoassay, EIA

Zur Bestimmung des surface Antigens (HBsAg) von HBV im Blut des Patienten aus Berlin wurde der Enzymimmunoassay Enzygnost® HBsAg 5.0 der Fa. Dade Behring Marburg GmbH, Marburg (Deutschland) angewendet.

Es handelt sich um einen in Europa zugelassenen leistungsfähigen Test, der den Angaben der Packungsbeilage gemäß abgearbeitet wurde.

Das zu Grunde liegende Testprinzip ist ein sogenannter Sandwich Test im Mikrotitrationsplatten-Format:
100 µl der zu untersuchenden Probe werden in einem Einschritt-Verfahren mit 25 µl Konjugat 1 (monoklonale HBsAg spezifische Antikörper von der Maus, die kovalent mit Biotin markiert sind) und immobilisierten HBsAg-spezifischen polyklonalen Antikörpern vom Schaf in Kontakt gebracht. Nach 60-minütiger Inkubation bei 37°C und Entfernen überschüssiger Komponenten durch 4-maliges Waschen der Platten-Kavitäten werden 100 µl Konjugat 2 zugegeben, das aus Streptavidin besteht, an das das Sonden-Enzym Peroxidase kovalent gebunden ist.
Nach 30-minütiger Inkubation bei 37°C und Entfernen überschüssiger Komponenten durch 4-maliges Waschen der Platten-Kavitäten werden 75 µl Chromogen-Puffer/Substrat-Lösung zugegeben, gefolgt von einer 30-minütigen Inkubation bei Raumtemperatur. Die Entwicklung des blau gefärbten Tetramethylbenzidin-Farbstoffes wird durch Zugabe von 75 µl Stoplösung (Schwefelsäure) unterbrochen und der Farbstoff bei 450 nm photometrisch gemessen.

Die Intensität der Farbentwicklung, gemessen an der optischen Dichte (O.D.) ist dem Gehalt der Untersuchungsprobe an HBsAg direkt proportional, wobei ein O.D. Wert (Lichtabsorptionswert), der niedriger als ein Grenzwert ist, als HBsAg-negativ bewertet wird. Der Grenzwert ist definiert als der O.D.-Mittelwert der parallel getesteten Kontrolle, negativ (im Testkit enthalten), zu dem ein konstanter Betrag von 0,05 O.D. addiert wird.

Die Nachweisgrenzen des zur Untersuchung herangezogenen Lots (# 35214) wurden mit den international akzeptierten Standardpräparationen des Paul-Ehrlich-Institutes, Langen, Deutschland zu 0,007 ng ad-Subtyp/ ml bzw. 0,005 ng ay-Subtyp / ml aus Testungen von Verdünnungen der Standardpräparationen in HBsAg-negativem Serum durch graphische Interpolation ermittelt.

Die Untersuchung der Probe # 126734 / 305024817, aus der auch die DNA-Isolierung vorgenommen wurde, brachte in 2 unabhängigen Versuchen an zwei verschiedenen Tagen Ergebnisse zwischen 0,02 und 0,03 O.D., die den Kriterien des Testes entsprechend als HBsAg-negativ zu interpretieren sind. Die mitgeführte Kontrolle, positiv (im Testkit enthalten) war positiv (Validierungskriterien erfüllt).

### Beispiel 2: Isolierung der HDB 07- DNA aus Probe # 126734 / 305024817

Ein 65 µl-Aliquot der Berliner Probe wurde mit 135µl negativem Plasma auf ein Volumen von 200µl aufgefüllt und die DNA isoliert, indem der QIA amp® Min Elute^{™} Virus Spin Kit der Fa. Qiagen, Hilden (Deutschland) angewendet wurde. Dabei wurden alle Verfahrensschritte wie in der Packungsbeilage beschrieben befolgt und die Elution in einem Volumen von je 20 µl vorgenommen.

### Beispiel 3: Polymerase Ketten Reaktion, PCR

### 2.1. HBV Primer

Die vier nachstehenden HBV Primer wurden verwendet:

| | |
|---|---|
| Primer 1 mit der 5'> 3'- Sequenz: | |
| GGGTCACCATATTCTTGGGAAC | (SEQ ID NO:28) |
| | |
| Primer 2 mit der 5'> 3'- Sequenz: | |
| TATACCCAAAGACAAAAGAAAATTGG | (SEQ ID NO:29) |
| | |
| Primer 3 mit der 5'> 3'- Sequenz: | |
| GACTCGTGGTGGACTTCTCTC | (SEQ ID NO:30) |
| | |
| Primer 4 mit der 5'> 3'- Sequenz: | |
| TACAGACTTGCCCCCCAATACC | (SEQ ID NO:31) |

### 2.2. PCR-Amplifikation

Es wurde eine sogenannte nested PCR amplification des surface Antigens durchgeführt, wobei der Perkin Elmer Ampli Taq ® DNA Polymerase Kit sowie der Thermocycler Gene Amp ® PCR system 9700 der Fa. Perkin Elmer Applied Biosystems, USA verwendet wurde.

Die Nukleotide wurden von der Fa. Amersham Biosciences, UK bezogen.

Für den ersten Amplifikations-Zyklus wurden 7 µl der isolierten DNA unter Verwendung der oben genannten Primer 1 und 2 sowie folgenden Bedingungen amplifiziert:

### PCR 1 rxn

| | |
|---|---|
| Primer 1 (10 µM) | 1 µl |
| Primer 2 (10 µM) | 1 µl |
| 10 -fach konz. Puffer (incl. 15 mM Mg2Cl) | 5 µl |
| dNTP Mischung (10 mM) | 1 µl |
| dest. Wasser | 34,75 µl |
| Ampli Taq (5 U/ µl) | 0,25 µl |
| Pro Röhrchen | 43 µl Gesamtvolumen |
| plus | 7 µl isolierte DNA |
| | 50 µl Reaktionsvolumen |

Der 50 µl-Ansatz wurde unter Verwendung des beschriebenen Thermocyclers unter folgenden Bedingungen amplifiziert:
94° C, 1 min. / 94°C, 28 sek. - 50° C, 28 sek. - 72 ° C, 60 sek. (40 cycles) / 72 ° C, 5 min. / 8 °C soak.
In der zweiten Amplifzierungsrunde wurde 7 µl des ersten PCR Produktes weiter amplifiziert unter Verwendung der HBV Primer 3 und 4 und folgenden Bedingungen:

### PCR 2 rxn

| | |
|---|---|
| Primer 3 (10 µM) | 1 µl |
| Primer 4 (10 µM) | 1 µl |
| 10 -fach konz. Puffer | 5 µl |
| dNTP Mischung (10 mM) | 1 µl |
| dest. Wasser | 34,75 µl |
| Ampli Taq (5 U/ µl) | 0,25µl |
| Pro Röhrchen | 43 µl Gesamtvolumen |
| plus | 7 µl PCR Produkt v.rxn |
| | 50 µl Reaktionsvolumen |

Dieser PCR 2- Ansatz wurde unter Verwendung des oben beschriebenen Thermocyclers amplifiziert wobei folgende Bedingungen angewendet wurden:
94° C, 1 min. / 94°C, 28 sek. - 55° C, 28 sek. - 72 ° C, 38 sek. (40 cycles) / 72 ° C, 5 min. / 8 °C soak.
Abschließend wurde das PCR 2 Produkt elektrophoretisch aufgetrennt (1,5 % Agarose) unter Mitführen geeigneter Molekulargewichtsmarker. Das PCR-Produkt (mit ca. 520 Basenpaaren) wurde mit Hilfe des QIA quick PCR Purification Kit der Fa. Qiagen, Hilden (Deutschland) aufgereinigt

### Beispiel 4: Sequenzierung von HDB 07

Das gereinigte PCR Produkt wurde von der Fa. Eurofin Medigenomix GmbH Martinsried (Deutschland) mit Hilfe des ABI 3700 Kapillar Systems sequenziert in Verbindung mit der ABI BigDye Terminator Chemistry Version 3.1. und der ABI Sequencing Analysis Software Version 3.7. unter Verwendung der in Bsp. 3 beschriebenen Primer 3 und 4.

### Sequenzierungs-Ergebnis

Es konnte gezeigt werden, daß das HBsAg der analysierten Probe innerhalb des sequenzierten Bereiches die beste Übereinstimmung der Nukleotid- und Aminosäure-Sequenz mit dem Genotyp D, Subtyp ayw2 zeigt. In der Region der a-Determinante zeigt die analysierte Probe aus Berlin insgesamt 7 Aminosäure-Substitutionen im Vergleich zum Genotyp D, Subtyp ayw2 (siehe auch Abb. 6):

| | **HDB 07:** | | **D, ayw2:** |
|---|---|---|---|
| 1) | Arg (R) | statt | 105 Pro (P) |
| 2) | Arg (R) | statt | 118 Thr (T) |
| 3) | Leu (L) | statt | 120 Pro (P) |
| 4) | Lys (K) | statt | 122 Arg (R) |
| 5) | Leu (L) | statt | 142 Pro (P) |
| 6) | Asn (N) | statt | 160 Lys (K) |
| 7) | Pro (P) | statt | 173 Leu (L) |
| 8) | Leu (L) | statt | 110 Ile (I) |

Zusätzlich liegt eine Aminosäure-Substitution in der Position # 100 vor:

| | | | |
|---|---|---|---|
| 9) | Tyr (Y) | statt | 100 Cys (C). |

## Patentansprüche

1. Oligo- oder Polypeptid umfassend
(a) eine Aminosäuresequenz, die eine Teilsequenz von SEQ ID NO:3 mit wenigstens 75 aufeinanderfolgenden Aminosäuren von SEQ ID NO:3 ist, wobei diese Teilsequenz alle acht der Positionen 100, 105, 110, 118, 120, 142, 160 und 173 von SEQ ID NO:3 einschließt; oder
(b) ein Fragment eines HBs-Antigens eines Hepatitis B-Virus, wobei das Fragment eine Länge von wenigstens 15 Aminosäuren hat, das HBs-Antigen an Position 100 Cystein, an Position 105 Arginin, an Position 110 Leucin, an Position 118 Arginin, an Position 120 Leucin, an Position 142 Leucin, an Position 160 Asparagin und an Position 173 Prolin aufweist, und das Fragment Cystein 100, Arginin 105, Arginin 118, Leucin 120, Leucin 142, Asparagin 160 und Prolin 173 Umfaßt.

2. Oligo- oder Polypeptid nach Anspruch 1, **dadurch gekennzeichnet, daß** es mit Seren aus Individuen reagiert, die mit der Hepatitis B-Virusvariante, umfassend eine Nukleotidsequenz, kodierend für eine Aminosäuresequenz, welche die SEQ ID NO:4 einschließt, infiziert sind.

3. Oligo- oder Polypeptid nach Anspruch 1 oder 2, **dadurch gekennzeichnet, daß** es eine Aminosäuresequenz umfaßt, die ausgewählt ist aus der Gruppe bestehend aus SEQ ID NO:3, SEQ ID NO:4 und SEQ ID NO:5.

4. Oligo- oder Polynukleotid, umfassend eine Nukleotidsequenz, die für ein Oligo- öder Polypeptid nach einem der Ansprüche 1 bis 3 kodiert oder ein dazu komplementäres Oligo- oder Polynukleotid.

5. Oligo- oder Polynukleotid nach Anspruch 4, **dadurch gekennzeichnet, daß** es eine Nukleotidsequenz bestehend aus SEQ ID NO:2 umfaßt.

6. Vektor oder Plasmid enthaltend ein Oligo- oder Polynukleotid nach einem der Ansprüche 4 bis 5.

7. Zelle, die mit einem Vektor oder Plasmid nach Anspruch 6 transformiert oder transfiziert wurde.

8. Zelle, die ein Oligo- oder Polynukleotid nach einem der Ansprüche 4 bis 5 oder einen Vektor oder ein Plasmid nach Anspruch 6 enthält.

9. Verfahren zur Herstellung eines Oligo- oder Polypeptids nach einem der Ansprüche 1 bis 3, das umfaßt, daß man eine Zelle nach Anspruch 7 oder 8 unter geeigneten Bedingungen kultiviert, so daß das Oligo- oder Polypeptid exprimiert wird.

10. Verfahren nach Anspruch 9, **dadurch gekennzeichnet, daß** das Oligo- oder Polypeptid aus den Zellen gewonnen wird und von anderen Oligo- oder Polypeptiden abgetrennt wird.

11. Testkit zum Nachweis von Hepatitis B-Viren, enthaltend ein Oligo- oder Polypeptid nach einem der Ansprüche 1 bis 3.

12. Immunogenes Peptid oder Mischung immunogener Peptide, enthaltend ein oder mehrere Oligo- oder Polypeptide nach einem der Ansprüche 1 bis 3 alleine oder in Verbindung mit bekannten HBV-Immunogenen.

13. Verfahren zum Nachweis von Antikörpern, die gegen ein Hepatitis B-Virus-Antigen gerichtet sind, **dadurch gekennzeichnet, daß**
(a) eine Probe mit einem Oligo- oder. Polypeptid nach einem der Ansprüche 1 bis 3 unter Bedingungen inkubiert wird, die die Bildung eines Antigen-Antikörper-Komplexes gestatten; und
(b) der Antikörper-Antigen-Komplex, der das Oligo- oder Polypeptid enthält, nachgewiesen wird.

14. Isoliertes Hepatitis B-Virus, das ein HBs-Antigen aufweist, das die Aminosäuresequenz SEQ ID NO:5 umfaßf.

## Claims

1. An oligo- or polypeptide comprising
(a) an amino acid sequence which is a partial sequence of SEQ ID No: 3 having at least 75 consecutive amino acids of SEQ ID No: 3, where this partial sequence includes all eight of positions 100, 105, 110, 118, 120, 142, 160 and 173 of SEQ ID No: 3; or
(b) a fragment of an HBs antigen of a hepatitis B virus, where the fragment has a length of at least 15 amino acids, the HBs antigen has cysteine at position 100, arginine at position 105, leucine at position 110, arginine at position 118, leucine at position 120, leucine at position 142, asparagine at position 160 and proline at position 173, and the fragment comprises cysteine 100, arginine 105, arginine 118, leucine 120, leucine 142, asparagine 160 and proline 173.

2. An oligo- or polypeptide as claimed in claim 1, which reacts with sera from individuals infected by the hepatitis B virus variant, comprising a nucleotide sequence coding for an amino acid sequence which includes SEQ ID No: 4.

3. An oligo- or polypeptide as claimed in claim 1 or 2, which comprises an amino acid sequence which is selected from the group consisting of SEQ ID No: 3, SEQ ID No: 4 and SEQ ID No: 5.

4. An oligo- or polynucleotide comprising a nucleotide sequence which codes for an oligo- or polypeptide as claimed in any of claims 1 to 3 or an oligo- or polynucleotide complementary thereto.

5. An oligo- or polynucleotide as claimed in claim 4, which comprises a nucleotide sequence consisting of SEQ ID No: 2.

6. A vector or plasmid comprising an oligo- or polynucleotide as claimed in any of claims 4 to 5.

7. A cell which has been transformed or transfected with a vector or plasmid as claimed in claim 6.

8. A cell which comprises an oligo- or polynucleotide as claimed in any of claims 4 to 5 or a vector or a plasmid as claimed in claim 6.

9. A method for preparing an oligo- or polypeptide as claimed in any of claims 1 to 3, which comprises culturing a cell as claimed in claim 7 or 8 under suitable conditions such that the oligo- or polypeptide is expressed.

10. The method as claimed in claim 9, wherein the oligo- or polypeptide is obtained from the cells and is separated from other oligo- or polypeptides.

11. An assay kit for detecting hepatitis B viruses, comprising an oligo- or polypeptide as claimed in any of claims 1 to 3.

12. An immunogenic peptide or mixture of immunogenic peptides comprising one or more of the oligo- or polypeptides as claimed in any of claims 1 to 3 alone or in combination with known HBV immunogens.

13. A method for detecting antibodies directed against a hepatitis B virus antigen, which comprises
(a) incubating a sample with an oligo- or polypeptide as claimed in any of claims 1 to 3 under conditions which allow the formation of an antigen-antibody complex; and
(b) detecting the antibody-antigen complex which comprises the oligo- or polypeptide.

14. An isolated hepatitis B virus which includes an HBs antigen which comprises the amino acid sequence SEQ ID No: 5.

## Revendications

1. Oligopeptide ou polypeptide comprenant
(a) une séquence d'acides aminés, qui est une séquence partielle de l'identification de séquence N°3 ayant au moins 75 acides aminés successifs de l'identification de séquence N°3, cette séquence partielle incluant toutes les huit des positions 100, 105, 110, 118, 120, 142, 160 et 173 de l'identification de séquence N°3 ou
(b) un fragment d'un antigène HBs d'un virus de l'hépatite B, le fragment ayant une longueur d'au moins 15 acides aminés, l'antigène d'HBs comprenant à la position 100 de la cystéine, à la position 105 de l'arginine, à la position 110 de la leucine, à la position 118 de l'arginine, à la position 120 de la leucine, à la position 142 de la leucine, à la position 160 de l'asparagine et à la position 173 de la proline, et le fragment comprenant en 100 de la cystéine, en 105 de l'arginine, en 118 de l'arginine, en 120 de la leucine, en 142 de la leucine, en 160 de l'asparagine et en 173 de la proline.

2. Oligopeptide ou polypeptide suivant la revendication 1, **caractérisé en ce qu'**il réagit sur des sérums d'individus, qui sont infectés par la variante du virus de l'hépatite B, comprenant une séquence de nucléotides, codant pour une séquence d'acides aminés, qui inclut l'identification de séquence N°4.

3. Oligopeptide ou polypeptide suivant la revendication 1 ou 2, **caractérisé en ce qu'**il comprend une séquence d'acides aminés, qui est choisie dans le groupe constitué de l'identification de séquence N°3, de l'identification de séquence N°4 et de l'identification de séquence N°5.

4. Oligonucléotide ou polynucléotide, comprenant une séquence de nucléotides, qui code pour un oligopeptide ou un polypeptide suivant l'une des revendications 1 à 3 ou un oligonucléotide ou polynucléotide qui en est complémentaire.

5. Oligonucléotide ou polynucléotide suivant la revendication 4, **caractérisé en ce qu'**il comprend une séquence de nucléotides constituée de l'identification de séquence N°2.

6. Vecteur ou plasmide contenant un oligonucléotide ou un polynucleotide suivant l'une des revendications 4 à 5.

7. Cellule, qui a été transformée ou transfectée par un vecteur ou par un plasmide suivant la revendication 6.

8. Cellule, qui contient un oligonucléotide ou un polynucléotide suivant l'une des revendications 4 à 5 ou un vecteur ou un plasmide suivant la revendication 6.

9. Procédé de préparation d'un oligopeptide ou d'un polypeptide suivant l'une des revendications 1 à 3, qui comprend que l'on cultive une cellule suivant la revendication 7 ou 8 dans des conditions appropriées, de manière à exprimer l'oligopeptide ou le polypeptide.

10. Procédé suivant la revendication 9, **caractérisé en ce que**, l'on obtient l'oligopeptide ou le polypeptide à partir des cellules et on le sépare d'autres oligopeptides ou d'autres polypeptides.

11. Trousse d'essai pour la détection de virus de l'hépatite B, contenant un oligopeptide ou un polypeptide suivant l'une des revendications 1 à 3.

12. Peptide immunogène ou mélange de peptides immunogènes, contenant un ou plusieurs oligopeptides ou polypeptides suivant l'une des revendications 1 à 3, seuls ou en liaison avec des immunogènes d'HBV connus.

13. Procédé de détection d'anticorps qui sont dirigés contre un antigène du virus de l'hépatite B, **caractérisé en ce que**
(a) on fait incuber un échantillon ayant un oligopeptide ou un polypeptide suivant l'une des revendications 1 à 3, dans des conditions qui permettent la formation d'un complexe antigènes-anticorps et
(b) on détecte le complexe anticorps-antigènes, qui contient l'oligopeptide ou le polypeptide.

14. Virus de l'hépatite B isolé, qui a un antigène d'HBs, comprenant la séquence d'acides aminés de l'identification de séquence N°5.


### SEQUENCE LISTING

<110> Siemens Healthcare Diagnostics Products GmbH
<120> Neue Oberflächen-Protein-Mutante des Hepatitis B Virus HBV surface Antigen (HBsAg)
<130> 2007/B008 - Ma 1292
<140> DE 10 2007 062 962.3
   <141> 2007-12-21
<160> 31
<170> PatentIn version 3.3
<210> 1
   <211> 678
   <212> DNA
   <213> Hepatitis B virus
<400> 1
   atggagaaca tcacatcagg attcctagga cccctgctcg tgttacaggc ggggtttttc      60
   ttgttgacaa gaatcctcac aataccgcag agtctagact cgtggtggac ttctctcaat     120
   tttctagggg gaactaccgt gtgtcttggc caaaattcgc agtccccaac ctccaatcac     180
   tcaccaacct cctgtcctcc aacttgtcct ggttatcgct ggatgtgtct gcggcgtttt     240
   atcatcttcc tcttcatcct gctgctatgc ctcatcttct tgttggttct tctggactgt     300
   caaggtatgt tacgcgtttg tcctctactt ccaggatctt caaccaccag caggggacta     360
   tgcaaaacct gcacgactcc tgctcaagga acctctatgt atccctcctg ttgctgtacc     420
   aaactttcgg acggaaattg cacctgtatt cccatcccat catcctgggc tttcggaaac     480
   ttcctatggg agtgggcctc agcccgtttc tcctggccca gtttactagt gccatttgtt     540
   cagtggttcg tagggctttc ccccactgtt tggctttcag ttatatggat gatgtggtat     600
   tgggggccaa gtctgtacag catcttgagt ccctttttac cgctgttacc aattttcttt     660
   tgtctttggg tatacatt                                                   678
<210> 2
   <211> 243
   <212> DNA
   <213> Hepatitis B virus
<400> 2
   tgtcaaggta tgttacgcgt ttgtcctcta cttccaggat cttcaaccac cagcagggga      60
   ctatgcaaaa cctgcacgac tcctgctcaa ggaacctcta tgtatccctc ctgttgctgt     120
   accaaacttt cggacggaaa ttgcacctgt attcccatcc catcatcctg ggctttcgga     180
   aacttcctat gggagtgggc ctcagcccgt ttctcctggc ccagtttact agtgccattt     240
   gtt                                                                   243
<210> 3
   <211> 226
   <212> PRT
   <213> Hepatitis B virus
<400> 3
   Met Glu Asn Ile Thr Ser Gly Phe Leu Gly Pro Leu Leu Val Leu Gln
   1               5                   10                  15
   Ala Gly Phe Phe Leu Leu Thr Arg Ile Leu Thr Ile Pro Gln Ser Leu
               20                  25                  30
   Asp Ser Trp Trp Thr Ser Leu Asn Phe Leu Gly Gly Thr Thr Val Cys
           35                  40                  45
   Leu Gly Gln Asn Ser Gln Ser Pro Thr Ser Asn His Ser Pro Thr Ser
       50                  55                  60
   Cys Pro Pro Thr Cys Pro Gly Tyr Arg Trp Met Cys Leu Arg Arg Phe
   65                  70                  75                  80
   Ile Ile Phe Leu Phe Ile Leu Leu Leu Cys Leu Ile Phe Leu Leu Val
                   85                  90                  95
   Leu Leu Asp Cys Gln Gly Met Leu Arg Val Cys Pro Leu Leu Pro Gly
               100                 105                 110
   Ser Ser Thr Thr Ser Arg Gly Leu Cys Lys Thr Cys Thr Thr Pro Ala
           115                 120                 125
   Gln Gly Thr Ser Met Tyr Pro Ser Cys Cys Cys Thr Lys Leu Ser Asp
       130                 135                 140
   Gly Asn Cys Thr Cys Ile Pro Ile Pro Ser Ser Trp Ala Phe Gly Asn
   145                 150                 155                 160
   Phe Leu Trp Glu Trp Ala Ser Ala Arg Phe Ser Trp Pro Ser Leu Leu
                   165                 170                 175
   Val Pro Phe Val Gln Trp Phe Val Gly Leu Ser Pro Thr Val Trp Leu
               180                 185                 190
   Ser Val Ile Trp Met Met Trp Tyr Trp Gly Pro Ser Leu Tyr Ser Ile
           195                 200                 205
   Leu Ser Pro Phe Leu Pro Leu Leu Pro Ile Phe Phe Cys Leu Trp Val
       210                 215                 220
   Tyr Ile
   225
<210> 4
   <211> 91
   <212> PRT
   <213> Hepatitis B virus
<400> 4
   Cys Gln Gly Met Leu Arg Val Cys Pro Leu Leu Pro Gly Ser Ser Thr
   1               5                   10                  15
   Thr Ser Arg Gly Leu Cys Lys Thr Cys Thr Thr Pro Ala Gln Gly Thr
               20                  25                  30
   Ser Met Tyr Pro Ser Cys Cys Cys Thr Lys Leu Ser Asp Gly Asn Cys
           35                  40                  45
   Thr Cys Ile Pro Ile Pro Ser Ser Trp Ala Phe Gly Asn Phe Leu Trp
       50                  55                  60
   Glu Trp Ala Ser Ala Arg Phe Ser Trp Pro Ser Leu Leu Val Pro Phe
   65                  70                  75                  80
   Val Gln Trp Phe Val Gly Leu Ser Pro Thr Val
                   85                  90
<210> 5
   <211> 81
   <212> PRT
   <213> Hepatitis B virus
<400> 5
   Cys Gln Gly Met Leu Arg Val Cys Pro Leu Leu Pro Gly Ser Ser Thr
   1               5                   10                  15
   Thr Ser Arg Gly Leu Cys Lys Thr Cys Thr Thr Pro Ala Gln Gly Thr
               20                  25                  30
   Ser Met Tyr Pro Ser Cys Cys Cys Thr Lys Leu Ser Asp Gly Asn Cys
           35                  40                  45
   Thr Cys Ile Pro Ile Pro Ser Ser Trp Ala Phe Gly Asn Phe Leu Trp
       50                  55                  60
   Glu Trp Ala Ser Ala Arg Phe Ser Trp Pro Ser Leu Leu Val Pro Phe
   65                  70                  75                  80
   Val
<210> 6
   <211> 5
   <212> PRT
   <213> Hepatitis B virus
<400> 6
   Ser Thr Thr Ser Arg
   1 5
<210> 7
   <211> 5
   <212> PRT
   <213> Hepatitis B virus
<400> 7
   Thr Thr Ser Arg Gly
   1 5
<210> 8
   <211> 5
   <212> PRT
   <213> Hepatitis B virus
<400> 8
   Thr Ser Arg Gly Leu
   1 5
<210> 9
   <211> 5
   <212> PRT
   <213> Hepatitis B virus
<400> 9
   Ser Arg Gly Leu Cys
   1 5
<210> 10
   <211> 5
   <212> PRT
   <213> Hepatitis B virus
<400> 10
   Arg Gly Leu Cys Lys
   1 5
<210> 11
   <211> 5
   <212> PRT
   <213> Hepatitis B virus
<400> 11
   Gly Leu Cys Lys Thr
   1 5
<210> 12
   <211> 5
   <212> PRT
   <213> Hepatitis B virus
<400> 12
   Leu Cys Lys Thr Cys
   1 5
<210> 13
   <211> 5
   <212> PRT
   <213> Hepatitis B virus
<400> 13
   Trp Ala Phe Gly Asn
   1 5
<210> 14
   <211> 5
   <212> PRT
   <213> Hepatitis B virus
<400> 14
   Ala Phe Gly Asn Phe
   1 5
<210> 15
   <211> 5
   <212> PRT
   <213> Hepatitis B virus
<400> 15
   Phe Gly Asn Phe Leu
   1 5
<210> 16
   <211> 5
   <212> PRT
   <213> Hepatitis B virus
<400> 16
   Gly Asn Phe Leu Trp
   1 5
<210> 17
   <211> 5
   <212> PRT
   <213> Hepatitis B virus
<400> 17
   Asn Phe Leu Trp Glu
   1 5
<210> 18
   <211> 12
   <212> DNA
   <213> Hepatitis B virus
<400> 18
   agcaggggac ta 12
<210> 19
   <211> 18
   <212> DNA
   <213> Hepatitis B virus
<400> 19
   agcaggggac tatgcaaa 18
<210> 20
   <211> 24
   <212> DNA
   <213> Hepatitis B virus
<400> 20
   agcaggggac tatgcaaaac ctgc 24
<210> 21
   <211> 45
   <212> DNA
   <213> Hepatitis B virus
<400> 21
   agcaggggac tatgcaaaac ctgcacgact cctgctcaag gaacc 45
<210> 22
   <211> 81
   <212> DNA
   <213> Hepatitis B virus
<400> 22
   agcaggggac tatgcaaaac ctgcacgact cctgctcaag gaacctctat gtatccctcc 60
   tgttgctgta ccaaactttc g 81
<210> 23
   <211> 12
   <212> DNA
   <213> Hepatitis B virus
<400> 23
   ttcggaaact tc 12
<210> 24
   <211> 18
   <212> DNA
   <213> Hepatitis B virus
<400> 24
   tgggctttcg gaaacttc 18
<210> 25
   <211> 27
   <212> DNA
   <213> Hepatitis B virus
<400> 25
   ccatcatcct gggctttcgg aaacttc 27
<210> 26
   <211> 48
   <212> DNA
   <213> Hepatitis B virus
<400> 26
   aattgcacct gtattcccat cccatcatcc tgggctttcg gaaacttc 48
<210> 27
   <211> 81
   <212> DNA
   <213> Hepatitis B virus
<400> 27
   ccctcctgtt gctgtaccaa actttcggac ggaaattgca cctgtattcc catcccatca 60
   tcctgggctt tcggaaactt c 81
<210> 28
   <211> 22
   <212> DNA
   <213> Hepatitis B virus
<400> 28
   gggtcaccat attcttggga ac 22
<210> 29
   <211> 26
   <212> DNA
   <213> Hepatitis B virus
<400> 29
   tatacccaaa gacaaaagaa aattgg 26
<210> 30
   <211> 21
   <212> DNA
   <213> Hepatitis B virus
<400> 30
   gactcgtggt ggacttctct c 21
<210> 31
   <211> 22
   <212> DNA
   <213> Hepatitis B virus
<400> 31
   tacagacttg gcccccaata cc 22